# EUROPEAN PATENT APPLICATION

(11) **EP 3 290 042 A2**
(43) Date of publication of application: **07.03.2018**
(21) Application number: 16789598.6
(22) Date of filing: 29.04.2016
(51) Int. Cl.: A61K 35/02, A61K 33/08, A61K 33/06, B01D 11/02, A61K 9/02

(54) **METHOD FOR PREPARING MULTIFUNCTIONAL NATURAL GEL AND NATURAL POWDER BY USING MINERAL-BASED SOMATIDS CONTAINED IN NATURAL MINERAL**

(30) Priority: 01.05.2015 KR 20150062279
(71) Applicant: Lee, Jong Doo, Uiwang-si, Gyeonggi-do 16046 (KR)
(72) Inventor: Lee, Jong Doo, Uiwang-si, Gyeonggi-do 16046 (KR)
(74) Representative: ABG Patentes, S.L.
(86) International application number: PCT/KR2016/004525
(87) International publication number: WO 2016/178492

(57) **Abstract**

The present invention provides a method for preparing a multifunctional natural gel by using mineral-based somatids extracted from a naturally produced mineral, comprising: (a) a powder reheating step of reheating mineral-based somatids at a predetermined high temperature in order to culture mineral-based somatids in the mineral-based somatids; (b) a mineral-based somatids separation step of mixing the mineral-based somatids heated in step (a) and water or distilled water in a predetermined ratio, and separating mineral-based somatids from the mineral-based somatids separated into an upper layer part, a middle layer part and a lower layer part through a stirrer and a centrifuge; and (c) a naturel gel preparation step of evaporating water or distilled water by heating the middle layer part separated in step (b) at a predetermined temperature, extracting only the remaining pure mineral-based somatid, and then preparing a gel-type natural gel from the extracted pure mineral-based somatid.

## Description

### I. Technical Field

The present invention relates to a method for manufacturing a multi-functional natural gel by using natural minerals and for manufacturing natural powder by using the natural gel and a method for manufactureing highly sophisticated materials for a variety of industrial fields by using the multi-functional natural gel and natural powder. More particularly, the present invention relates to a method for manufacturing multi-functional natural gel and natural powder using mineral somatid contained in natural minerals, in which mineral somatid that exists in natural minerals is separated and multi-functional natural gel and natural powder that contain a high concentration of mineral somatid are manufactured. When the multi-functional natural gel or natural powder is added at a certain ratio into metal materials, macromolecular materials, ceramic materials, food materials, cosmetic materials, anti-cancer drugs, immunity-enhancing drugs, anti-diabetic drugs, antihypotensive drugs, dermatologic drugs, and pharmaceutical raw materials, the effect of immunological enhancement, cancer cell inhibition, and skin regeneration, etc., can be endowed to the existing materials.

### II. Background Art

Researches on somatid, which is understood as being an intermediate between objects and life bodies, have been made from 150 years ago to the present, pioneered by Bechamp and continued by Enderlein, Reich, Reif, and Gastong Nesang, etc.

However, most of the researches centered on somatid in living bodies, and focused on revealing the biological characteristics of somatid. Nevertheless, according to a report from Bechamp, microzyma, a sort of tiny microbes that exist in the blood in human bodies and serve as the third element of blood, is within an intermediate range between objects and life bodies, and is the cause for fermentation. Furthermore, microzyma not only exists in life bodies, but also is observed in our surrounding environment, including soil.

Especially, fermentation will happen if calcium carbonate collected in the natural world is used, but will not happen if pure calcium carbonate in the same composition obtained through chemical synthesis is used. Based on that fact, it is believed that those tiny life bodies only exist in natural materials in the natural world. Through further research, it is found that those tiny life bodies not only exist in soil, but also exist in swamp, chimney soot, street dust, air, and water, etc. If the animal body that serves as the host of those tiny life bodies dies, the tiny life bodies will decompose the animal body and directly return to the nature. Owing to the effect of such tiny life bodies, though life bodies can exist, the decomposition of life bodies also becomes possible, and thereby circulation of materials of life bodies happens. It is proven that such tiny life bodies also existed in marbles before 6 million years when mammals began to emerge on the earth.

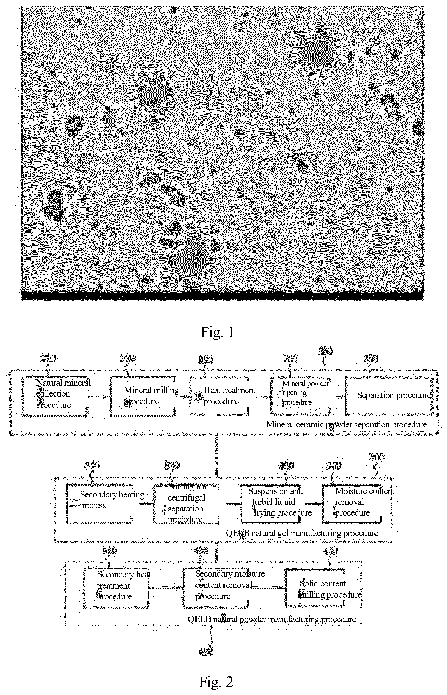

In addition, as shown in Fig. 1, recently, Gastong Nesang, who made research on the aforementioned tiny microbes (i.e., microzyma), observed tiny microbes with a high-magnification special microscope with magnification above 30,000x. He believed that the tiny microbes are a concrement of energy, which is an immortal being, and asserted that the tiny life bodies are an indispensable being in life phenomena, and named them as somatid.

Moreover, a method for obtaining somatid that can provide position effects to human bodies has been disclosed in published Japanese Patent No. 2006-166738 (Jun. 29, 2006).

In the prior patent, somatid is obtained with an ancient somatid collection method, which comprises the following procedures: processing shell fossil into particles in 0.1∼5µm particle size; mixing the shell fossil particles with water at a ratio of at least 5cc water per Ig shell fossil particles; keeping the mixed solution of the particles and water in still state for at least 4h; separating a precipitate that mainly contains calcium carbonate and a supernatant from the mixed solution that has been kept still for 4h or longer time, and taking the supernatant as water that contains ancient somatid.

However, in the prior art, the materials used for obtaining somatid are only limited to ancient shell fossil, and the quantity of obtained somatid is very low. Therefore, the somatid obtained with such a method may be used simply for a research purpose, it is difficult to use the somatid as a material required in actual life.

In addition, for the foresaid somatid, a group led by Professor Kwang-Sup Soh in Seoul National University has made research on a subject of the correlation between Bonghan ducts, vital granules and cell multiplication and published several articles recently. The subject of the research has some aspects similar to life bodies somatid that exist in blood and cerebrospinal fluid in human bodies, animals, and plants.

In the prior art, the research is made using somatid that exists in life bodies such as human bodies or animals and plants. However, owing to the characteristics of life bodies environment, it is difficult to culture and extract somatid in a large quantity, and the somatid cannot be applied widely in the industry. Therefore, the somatid obtained with such a method cannot be used in industry and as a material for industries of fusion/composite.

### III. Contents of the Invention

**Technical problem to be solved:** In order to solve the problem described above, an object of the present invention is to provide a method for manufacturing advanced materials using multi-functional natural gel and natural powder of mineral somatid, in which mineral somatid that exists in natural minerals is extracted at a high concentration and thereby multi-functional natural gel is manufactured and natural powder is manufactured using the natural gel, and the natural gel and natural powder are utilized through fusion/composition at a certain ratio or used separatly to manufacture metal materials, macromolecular materials, ceramic materials, food materials, cosmetic materials, pharmaceutical raw materials, anti-cancer drugs, immunity-enhancing drugs, anti-diabetic drugs, antihypotensive drugs, dermatologic drugs, and pharmaceutical raw materials. Thus, the effect of immunological enhancement, cancer cell inhibition, and skin regeneration etc., can be endowed to the existing materials.

Furthermore, an object of the present invention is to provide a method for manufacturing advanced materials using multi-functional natural gel and natural powder of mineral somatid, in which natural gel and natural powder that contains mineral somatid is extracted from mineral somatid, and the natural gel and natural powder are fused/composited with existing industrial materials at a certain ratio; thus, multi-functional advanced materials with such additional material functions for industries can be obtained more easily and conveniently, and can be produced in mass production at a low cost. The mineral somatid will not die even after it is heated at 1,000°C high temperature for 10h and precipitated for 2h in 36% hydrochloric acid; instead, the mineral somatid will still survive and emit energy.

Furthermore, an object of the present invention is to provide a method for manufacturing advanced materials using multi-functional natural gel and natural powder of mineral somatid. The mineral somatid can inhibit oxidation and induce reduction, enhance immunity of living bodies in a contactless manner, activate the metabolism of living bodies, and induce oxidation-resistant and aging-resistance effects.

Furthermore, an object of the present invention is to provide a method for manufacturing advanced materials using multi-functional natural gel and natural powder of mineral somatid, in which the mineral somatid is fused with common materials such as metal materials, macromolecular materials, and ceramic materials, etc., so that the material properties are improved.

Furthermore, an object of the present invention is to provide a method for manufacturing advanced materials using multi-functional natural gel and natural powder of mineral somatid, in which mineral somatid existing in natural minerals that only contain components including SiO₂, Al₂O₃, Fe₂O₃, MgO₃, and K, etc., is separated and extracted, and thereby the mineral somatid can be fused/composited with industrial materials at a certain ratio, such as metal materials including Au, Fe, Ag, Cu, Zn, stainless steel, and Ti; macromolecular materials including PBT, PET, PE nylon, epoxy resin, and silicon; and ceramic materials such as cement, carbon, and ferrite, etc.

**Technical solution to solve the technical problem:** In order to attain the objects described above, according to an embodiment of the present invention, the present invention provides a method for manufacturing rehabilitating multi-functional natural gel of quantum energy living body (QELBY) and natural powder of QELBY using mineral somatid powder extracted from minerals produced naturally. The method comprises: step of mineral collection: collecting minerals that contain components including SiO₂, Al₂O₃, Fe₂O₃, MgO₃, and K, etc., alone, or all of them; step of mineral milling: milling the minerals collected through the mineral collection step into 320-mesh or less mineral powder; step of heat treatment of residual organic substances: heating the mineral powder at high temperature of 200 °C to 1,000°C for 1∼2h, so as to remove impurities or residual organic substances; step of powder ripening: mixing the mineral powder after the heat treatment of residual organic substances with water or distilled water at a certain weight ratio, and ripening and fermenting at normal temperature; step of ceramic powder separation: removing moisture content from the mixture after ripening and fermentation, and drying the moisture-removing mixture at 180°C for 3h or longer time, so as to only separate mineral ceramic powder from the mixture; step of powder heating: reheating the ceramic powder at high temperature of 100 °C ∼1,000°C; step of mineral somatid separation: separating mineral somatid from the mineral ceramic powder after the reheating; step of natural gel manufacturing: heating and drying the mineral somatid at 100°C ∼600°C for 1h ∼10h so as to evaporate moisture content, distilled water, and deionized water, and then manufacturing the mineral somatid into natural gel in a gel form; and step of natural powder manufacturing: treating the natural gel by heat treatment, so as to manufacture the natural gel into natural powder.

Furthermore, according to an embodiment of the present invention, in the step of mineral collection, the minerals are natural minerals including loess, medical stone, jade, germanium, tourmaline, zeolite, kaolin, bentonite, and crystal.

Furthermore, according to an embodiment of the present invention, the minerals are milled with alumina ball mill (alumina rotary mill) and jet mill through either a dry process or a wet process.

Furthermore, according to an embodiment of the present invention, the step of mineral milling further comprises a step of mineral powder sorting, so that powders that contain SiO₂, Al₂O₃, Fe₂O₃, MgO₃, and K components alone or all of them are sorted out respectively from the mineral powder after the milling.

Furthermore, according to an embodiment of the present invention, in the step of powder ripening, 10∼80wt% of mineral powder is mixed with 20∼90wt% of water or distilled water to form a mixture, and then the mixture is stored in a specified container and ripened at 1∼100°C for 2∼60 days, and is fermented through the ripening.

Furthermore, according to an embodiment of the present invention, in the step of mineral ceramic powder separation, the mineral ceramic powder is separated from the mineral powder through a primary heating step and a secondary heating step, wherein, in the primary heating step, the ripened mixture is loaded into a roasting oven and rotated at 180°C or a lower temperature for 30min.∼2h to remove the moisture content, and then heating the mixture in a desiccator at 100∼200°C for 3h to accomplish drying; in the secondary heating step, which follows the primary heating step, the mixture is heated at high temperature of 400∼1,000°C to accomplish drying.

Furthermore, according to an embodiment of the present invention, the step of mineral somatid separation comprises: a stirring step: the mineral ceramic powder is mixed with water or distilled water or deionized water, and then the mixture is loaded into a stirring machine and stirred for 24h; a step of mineral somatid separation: after the stirring step is finished, the mixture is centrifuged in a centrifugal separator for 1h ∼24h, so as to accomplish centrifugal separation of the mineral ceramic powder and the mineral somatid; a step of layer separation: after the centrifugal separation is finished, precipitation is executed for 3h ∼72h, so as to accomplish layer separation of the mineral ceramic powder and water or distilled water or deionized water.

Furthermore, according to an embodiment of the present invention, in the step of layer separation, the mixture is separated into a top layer portion, a bottom layer portion, and a middle layer portion, wherein, the water or distilled water or deionized water is located in the top layer portion, the mineral ceramic powder that fully contains components including SiO₂, Al₂O₃, Fe₂O₃, MgO₃, K, etc., is located in the bottom layer portion, and the middle layer portion consists of a suspension and turbid liquid that contains mineral somatid, and the mineral somatid is separated by only collecting the middle layer portion that consists of the suspension and turbid liquid.

Furthermore, according to an embodiment of the present invention, the natural gel contains 40∼80wt% SiO₂, 10∼35wt% Al₂O₃, 5∼10wt% Fe₂O₃, 3∼7wt% MgO₃, and 2∼8wt% K alone, or all of them.

Furthermore, according to an embodiment of the present invention, in the step of manufacturing natural powder of mineral somatid, the natural powder of mineral somatid is manufactured through a primary heat treatment step and a secondary heat treatment step, wherein, in the primary heat treatment step, the natural gel is dried at a temperature of 100°C ∼800°C for 2h ∼20h; in the secondary heat treatment step, the natural gel is treated by high pressure sterilization at 121°C for 15min. in an autoclave.

Furthermore, according to an embodiment of the present invention, in the step of manufacturing natural powder of mineral somatid, after the secondary heat treatment step, the resultant solid content obtained through the heat treatment is further dried at a temperature of 100∼800°C for 2∼20h.

**Benefits:** According to the present invention, multi-functional natural gel and natural powder are manufactured using mineral somatid that exists in minerals, and thereby the multi-functional natural gel and natural powder can be fused/composited at a certain ratio with industrial materials, such as metal materials, including Au, Fe, Ag, Cu, Zn, stainless steel, and Ti; macromolecular materials, including PBT, PET, PE nylon, epoxy resin, and silicon; and ceramic materials such as cement, carbon, and ferrite, etc., so as to manufacture advanced fusion/composite materials, and to make a great contribution to the development of the world economy and increase of jobs.

Moreover, according to the present invention, an oxidation-resistant function can be provided. Specifically, the water in a living body can be structured under the effect of energy emitted in the life activities of mineral somatid, which has no harm or side effect to the living body, in a contactless manner through the living body or glass, so that the active oxygen is reduced by 5 times or more, and the pH is kept neutral.

Moreover, according to the present invention, by virtue of the energy emitted in the life activities of mineral somatid, not only cancer cell inhibition, immunological enhancement, and skin regeneration effects, etc., can be improved, but also body warm or cool feeling can be improved, VOC can be removed, and harmful electromagnetic wave and heat release can be screened and absorbed.

Moreover, according to the present invention, by virtue of the energy emitted in the vital activities of mineral somatid, indoor humidity and temperature control can be realized.

Moreover, according to the present invention, the fusion/composite oxidation-resistant and multi-functional advanced ceramic materials that contains high-dispersity mineral somatid can be used to manufacture drugs, bio-products, and foods for treating cancer, diabetes, hypotension, cerebral apoplexy, AIDS, Parkinson's disease, metabolic diseases, cardiac diseases, and organ transplantation, and thereby treat humans, animals and plants healthily.

Moreover, according to the present invention, the fusion/composite oxidation-resistant and multi-functional advanced ceramic materials that contain high-dispersity mineral somatid can be used to manufacture fusion/composite advanced materials related with nuclear fusion, nuclear-related facilities, radar wave shielding and absorbing, heat-dissipating coating for weapons, weapons for national defenses, and non-weapon equipment, as well as fusion/composite advanced materials for space-crafts, aircrafts, watercrafts, and vehicles, etc., and thereby make a great contribution to the development of world economy and increase of jobs.

Moreover, according to the present invention, the fusion/composite oxidation-resistant and multi-functional advanced ceramic materials that contain high-dispersity mineral somatid can be used to manufacture other building materials, such as cement, ceramic tiles, bricks, paint, heat insulating materials, flooring materials, and doors and windows, etc.; and agricultural materials such as soil conditioners, green fertilizers, pesticides, and plastic heat insulating materials, etc., and thereby make a great contribution to the development of world economy and increase of jobs.

Moreover, according to the present invention, the fusion/composite oxidation-resistant and multi-functional advanced ceramic materials that contain high-dispersity mineral somatid can be used to manufacture environment-friendly materials for improving water quality, improving green algae, purifying waste water, purifying toxic soot, improving albinism, and purifying oil and grease, etc., and thereby promote human health and animal and plant health.

Moreover, according to the present invention, the fusion/composite oxidation-resistant and multi-functional advanced ceramic materials that contain high-dispersity mineral somatid can be used to manufacture advanced fusion/composite materials for IT, electronics, semiconductors, LCDs, LEDs, and 3D products, etc., and thereby make a great contribution to the development of world economy and increase of jobs.

Moreover, according to the present invention, the fusion/composite oxidation-resistant and multi-functional advanced ceramic materials that contain high-dispersity mineral somatid can be used to manufacture high-polymer resin, fiber, costume, and chemical materials, and thereby provide costumes for life convenience.

Moreover, according to the present invention, the fusion/composite oxidation-resistant and multi-functional advanced ceramic materials that contain high-dispersity mineral somatid can be fused with macromolecular materials easily, such as fiber and chemical materials, etc., and can be developed into fusion/composite materials with other materials including ferrous materials, non-ferrous materials, and ceramic materials, for the entire industry.

Moreover, according to the present invention, cells or microbes that are beneficial for living bodies or have no adverse effect to living bodies are not attacked or killed; instead, those cells or microbes are protected to survive in high temperature or extreme environments, and pathogenic bacteria that are harmful to living bodies or super bacteria that produce drug resistance against antibiotics are inhibited (sterilized).

### IV. Description of Drawings

Fig. 1 is a schematic diagram of somatid contained in blood taken with a high-magnification phase contrast microscope at a magnification of 400x or high according to the prior art;
Fig. 2 is a block diagram of the mineral somatid extraction method and the manufacturing process of multi-functional natural gel and natural powder using the mineral somatid according to an embodiment of the present invention;
Fig. 3 is a flow diagram of the mineral ceramic powder separation process according to the present invention;
Fig. 4 is a flow diagram of the process of manufacturing oxidation-resistant natural gel and natural powder using mineral somatid according to an embodiment of the present invention;
Fig. 5 is a schematic diagram of mineral powder after drying according to an embodiment of the present invention;
Fig. 6 shows photos of extracted mineral somatid in Fig. 3 taken with a phase contrast electron microscope at a magnification of 400x and 1000x respectively according to an embodiment of the present invention;
Fig. 7 shows the result of FT-IR analysis of the top layer portion extracted with the method shown in Fig. 3 according to an embodiment of the present invention;
Fig. 8 shows the result of SEM of the top layer portion extracted with the method shown in Fig. 3 after drying according to an embodiment of the present invention;
Figs. 9 and 10 show the result of SEM of the middle layer portion extracted with the method shown in Fig. 3 after drying according to an embodiment of the present invention;
Fig. 11 shows the experimental result of primary culturing with the method shown in Fig. 3 according to an embodiment of the present invention;
Figs. 12-14 show the results of exploratory and functional analysis of the mineral somatid and microbial strains cultured through secondary culturing with the method shown in Fig. 3 according to an embodiment of the present invention;
Figs. 15-17 show the experimental result of efficacy of the mineral somatid and microbial strains cultured through secondary culturing with the method shown in Fig. 3 according to an embodiment of the present invention;
Figs. 18-20 show the experimental result of VOC and ammonia removal performance of the mineral somatid according to a preferred embodiment of the present invention;
Figs. 21 and 22 show the panel that contains mineral somatid for experiment of electromagnet wave absorption and shielding performance of the mineral somatid according to the present invention;
Figs. 23-25 show the experimental result of electromagnet wave absorption performance of the mineral somatid according to a preferred embodiment of the present invention;
Fig. 26 is a schematic diagram that shows the experimental result of electromagnet wave shielding performance of the mineral somatid according to a preferred embodiment of the present invention;
Fig. 27 is a schematic diagram that shows the experimental result of heat producing capability of the mineral somatid according to a preferred embodiment of the present invention;
Figs. 28-30 show the experimental result of self-current charging of the mineral somatid according to a preferred embodiment of the present invention;
Fig. 31 is a schematic diagram that shows the experimental result of zeta potential of the mineral somatid according to a preferred embodiment of the present invention;
Figs. 32 and 33 show the result of confirmation experiment of oxidation-resistant effect of the mineral somatid according to a preferred embodiment of the present invention;
Figs. 34 and 35 show the result of measuring the effect of quantity of generated biophoton on the prevention of tissue necrosis and anti-oxidation of the mineral somatid according to a preferred embodiment of the present invention by using a photomultiplier tube;
Figs. 36 and 37 show the chart and data sheet of result of re-verification of the released quantity of biophotons after holding for 16h initially;
Figs. 38 and 39 show the chart and data sheet of result of re-verification of the released quantity of biophotons after holding for 7 days;
Figs. 41-46 show the result of experiment of oxidation prevention efficacy of the mineral somatid according to a preferred embodiment of the present invention;
Figs. 47-49 show the result of experiment of energy production response of the multi-functional natural gel and natural powder according to a preferred embodiment of the present invention under irradiation of UV light and visible light;
Figs. 50-52 show the result of the produced water energy response of the multi-functional natural gel and solid content according to a preferred embodiment of the present invention under irradiation of UV light and visible light;
Figs. 53-56 show the result of the produced edible oil energy response of the natural powder of mineral somatid according to a preferred embodiment of the present invention under irradiation of UV light and visible light;
Fig. 57 shows a photo of the suspension and turbid liquid of mineral somatid according to a preferred embodiment of the present invention taken with a phase contrast electron microscope at 1,000x magnification;
Figs. 58-64 show the results of FT-IR, TGA, XRF, and XRD experiments of the multi-functional natural gel and natural powder according to a preferred embodiment of the present invention.

### V. Detailed Description of Embodiments

The present invention relates to a method for manufacturing multi-functional natural gel and natural powder using mineral somatid, which is a method for manufacturing rehabilitating multi-functional natural gel and natural powder of quantum energy living body (QELBY) using mineral somatid powder extracted from minerals produced naturally, comprising:
a step of mineral collection: collecting minerals that contain components including SiO₂, Al₂O₃, Fe₂O₃, MgO₃, and K, etc., alone, or all of them;
a step of mineral milling: milling the minerals collected through the mineral collection step into 320-mesh or less mineral powder;
a step of heat treatment of residual organic substances: heating the mineral powder at 200∼1,000°C high temperature for 1∼2h, so as to remove impurities or residual organic substances;
a step of powder ripening: mixing the resultant mineral powder after the heat treatment of residual organic substances with water or distilled water at a certain weight ratio, and ripening and fermenting at normal temperature;
a step of ceramic powder separation: removing moisture content from the mixture after ripening and fermentation, and drying the mixture at 180°C for 3h or longer time after the moisture content is removed, so as to only separate mineral ceramic powder from the mixture;
a step of powder heating: reheating the ceramic powder at 100∼1,000°C high temperature;
a step of mineral somatid separation: separating mineral somatid from the mineral ceramic powder after the reheating;
a step of natural gel manufacturing: heating and drying the mineral somatid at 100∼600°C temperature for 1∼10h so as to evaporate moisture content, distilled water, and deionized water, and then manufacturing the mineral somatid into natural gel in a gel form; and
a step of natural powder manufacturing: treating the natural gel by heat treatment, to manufacture the natural gel into natural powder.

### Embodiments

Hereunder some preferred embodiments of the present invention will be detailed with reference to the accompanying drawings. First, for the reference labels attached to the constituting elements in the accompanying drawings, it should be noted that the same constituting element is labeled with the same reference label as far as possible even in different accompanying drawings. Moreover, for the description of the present invention, the detailed description of relevant well-known constructions or functions is omitted if the specific description is judged as possibly confusing the subject matter of the present invention.

Hereunder some preferred embodiments of the present invention will be detailed with reference to the accompanying drawings. First, for the reference labels attached to the constituting elements in the accompanying drawings, it should be noted that the same constituting element is labeled with the same reference label as far as possible even in different accompanying drawings. Moreover, for the description of the present invention, the detailed description of relevant well-known constructions or functions is omitted if the specific description is judged as possibly confusing the subject matter of the present invention.

Only the mineral somatid that contains SiO₂, Al₂O₃, Fe₂O₃, MgO₃ and K in the present invention is extracted, and the mineral somatid is manufactured into natural gel in a gel form and natural powder, the natural gel and natural powder are fused/composited with industrial materials such as TV casing materials, semiconductor chip materials, copper wires, coating agents for heat dispersion, coating agent for electromagnetic wave shielding, and alloys or synthetic materials; thus, to produce fusion/composite oxidization-resistant materials for the entire industry in mass production at a low cost conveniently, the process shown in Figs. 2-4 is implemented.

Fig. 2 is a block diagram of the mineral somatid extraction method and the manufacturing process of multi-functional natural gel and natural powder using mineral somatid according to an embodiment of the present invention. The mineral ceramic powder separation process 200 is used to separate mineral ceramic powder contained in natural minerals, and includes implementation of the following procedures: a natural mineral collection procedure 210: collecting natural minerals that respectively contain components including SiO₂, Al₂O₃, Fe₂O₃, MgO₃, and K, etc., alone, or all of them; a mineral milling procedure 220: milling the collected natural minerals; a heat treatment procedure 230: treating the milled mineral powder by heat treatment; a mineral powder ripening and fermentation procedure 240: ripening and fermenting the mineral powder treated through the heat treatment procedure 230, and drying the fermented mineral powder; a separation procedure 250: in order to separate the mineral ceramic powder contained in the fermented mineral powder fermented through mineral powder ripening and fermentation procedure 240, heating the fermented mineral powder at a high temperature for a specific time, so as to separate mineral ceramic powder from the fermented mineral powder.

Furthermore, in order to manufacture natural gel from the mineral somatid manufactured through the mineral somatid manufacturing process 200, a manufacturing process 300 of natural gel that contains mineral somatid is executed. The natural gel manufacturing process 300 comprises the following procedures: a heating procedure 310: reheating the mineral ceramic powder; a stirring and centrifugal separation procedure 320: stirring the heated mineral ceramic powder and executing centrifugal separation, to separate mineral somatid from the mineral ceramic powder; a suspension and turbid liquid drying procedure 330: extracting suspension and turbid liquid utilizing a layer separation phenomenon of the mineral somatid treated through separation, and drying and heating the suspension and turbid liquid; a moisture content removing procedure 340: removing the moisture content contained in the suspension and turbid liquid after drying, so as to only collect mineral somatid in a gel state.

Furthermore, in order to manufacture the natural gel manufactured through the natural gel manufacturing process 300 into natural powder, a natural powder manufacturing process 400 is executed. The natural powder manufacturing process 400 comprises the following procedures: a secondary heat treatment procedure 410: treating the natural gel by heat treatment; a secondary moisture content removing procedure 420: removing moisture content from the natural gel treated through heat treatment; a solid content milling procedure 430: milling the solid content after the moisture content is removed.

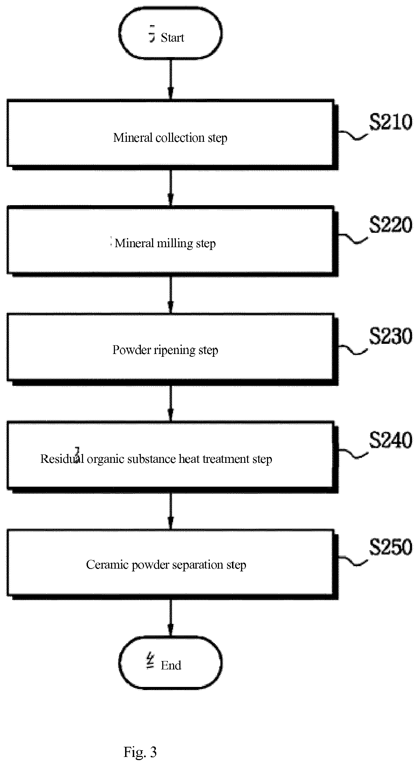

Fig. 3 is a flow diagram of the mineral ceramic powder separation process according to an embodiment of the present invention.

As shown in the figure, the mineral ceramic powder separation method in the present invention comprises a mineral collection step S210, a mineral milling step S220, a residual organic substance heat treatment step S230, a powder ripening step S240, and a ceramic powder separation step S250.

The mineral collection step S210 is a step executed through the natural mineral collection procedure 210, as well as a step in which the surface layer with plants growing healthily without moss or mold in a specific region is collected and then minerals that contain components including SiO₂, Al₂O₃, Fe₂O₃, MgO₃, and K, etc. alone, or all of them, are collected. It is also a step of collecting a mineral, which is well known from oriental medical and classic literatures, is referred to as healing stones or five colored stones and well known as having healing functions.

In such a mineral collection step S210, among natural minerals including loess, medical stones, jade, germanium, tourmaline, zeolite, kaolin, bentonite, and crystal, clay minerals in the feldspar series, which contain components including SiO₂, Al₂O₃, Fe₂O₃, MgO₃, and K, etc. alone, or all of them, are collected, and natural minerals that do not contain heavy metal or radioactive substances harmful to living bodies and are not contaminated are excavated from the land.

In the mineral milling step S220, the collected minerals are milled with alumina ball mill (alumina rotary mill) and jet mill through the mineral milling procedure 220 into 320-mesh or less mineral powder through either a dry process or a wet process.

Furthermore, preferably, a mineral powder sorting step is further executed after the milling of the mineral powder, but the process is not limited to that step. In the mineral powder sorting step, only powders that contain components including SiO₂, Al₂O₃, Fe₂O₃, MgO₃, and K, etc. alone, or all of them, respectively are sorted and extracted.

In the residual organic substance heat treatment step S230, the milled mineral powder is heated at 200∼1,000°C for 1∼2h through the heat treatment procedure 230, so as to remove impurities and residual organic substances. Through the residual organic substance heat treatment step, the agglomeration phenomenon of the mineral powder can be mitigated, and the fluidity of the mineral powder can be maximized.

In the powder ripening step S240, the mineral somatid that exists in a dormant state in the mineral powder is activated through the powder ripening procedure 240. The mineral powder is mixed with water or distilled water at a certain weight ratio, and the mixture is ripened and fermented at normal temperature for certain time.

In such a powder ripening step S240, 10∼80wt% mineral powder is mixed with 20∼90wt% water or distilled water to form a mixture, and then the mixture is stored in a specified container and ripened at a temperature of 1∼100°C for 2∼60 days and thereby fermented through the ripening process, i.e., the mineral powder is fermented naturally by virtue of its inherent components.

However, the present invention is not limited to that. Preferably, 75wt% mineral powder is mixed with 25wt% water or distilled water to form a mixture, and then the mixture is stored in a stainless steel container or ceramic container and ripened and fermented at normal temperature for at least 15 days in a fully sealed state.

Especially, the powder ripening step S240 in the present invention further maximizes the fermentation efficiency while accomplishing ripening, and it further includes extracts of roots, stems and leaves of natural plants (agar), which serve as zymocyte, to realize ripening and fermentation. In this case, preferably the quantity of the zymocyte does not exceed 5wt% of the total weight of the mineral powder and distilled water, to realize fermentation.

In the ceramic powder separation step S250, moisture content is removed from the ripened and fermented mixture through a mineral ceramic powder separation procedure S250, and the mixture is dried at 180°C for 3h or longer time after the moisture content is removed, so as to only separate mineral ceramic powder from the mixture.

In such a mineral ceramic powder separation step S250, the mineral ceramic powder is separated from the mineral powder through a primary heating step and a secondary heating step, wherein, in the primary heating step, the ripened mixture is loaded into a roasting oven and rotated at 180°C or a lower temperature for 30min.∼2h so as to remove moisture content, and then the mixture is heated in a desiccator at 100∼200°C temperature for 3h to accomplish drying; in the secondary heating step, the mixture is heated at high temperature of 400∼1,000°C to accomplish drying.

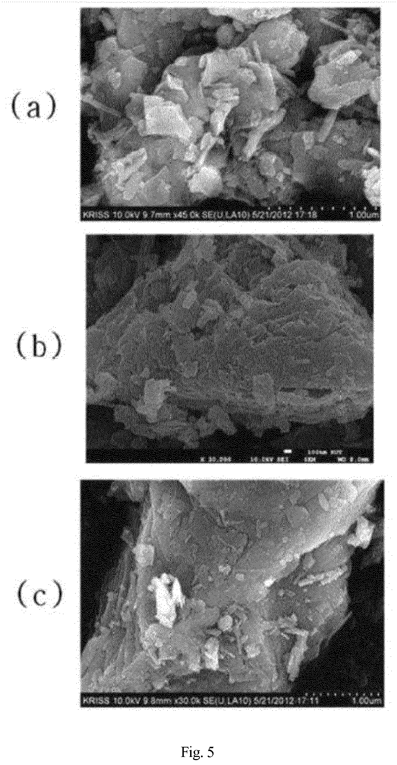

Fig. 5 shows the observation result of SEM of the surface of the mixture after ripening and drying, wherein, Figs. 5(a) and 5(b) show the observation result of the surface of the powder after ripening and primary drying, and Fig. 5(c) shows the observation result of the surface of the mixture after secondary drying at high temperature of 400∼1,000°C for 1h.

In other words, the mineral somatid described in the present invention exists in the above-mentioned ripened and dried mixture and is constitute of a form similar to micro impurities in the mineral ceramic powder; moreover, a step of further heating the mineral ceramic powder at 100∼1,000°C for 1h is executed, so as to confirm that a large quantity of mineral somatid, which have behaviors similar to those of somatid in living bodies, survives actively in the mineral ceramic powder.

In other words, in the present invention, in order to only separate pure mineral somatid from the mineral ceramic powder, the mineral ceramic powder is heated and dried at high temperature of 100∼1,000°C, so that all other organic substances except the mineral somatid are burnt off.

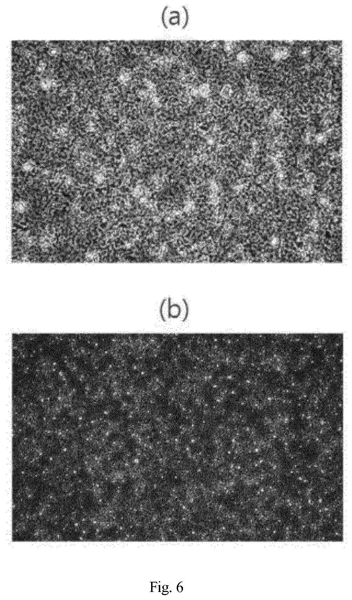

Especially, in the case shown in Fig. 5(c), the powder produced after the ripening is finished is heated at 100∼1,000°C for 1h, and then is observed under a phase contrast microscope. The result is shown in Fig. 6(a). It is confirmed that the mineral somatid is more uniform in shape and move actively even it is heated at such a high temperature. Fig. 6(b) shows a photo of the mineral somatid at magnification of 1,000x after the movement of the mineral somatid is fixed by adding oil into the powder for the convenience of observation at a high magnification.

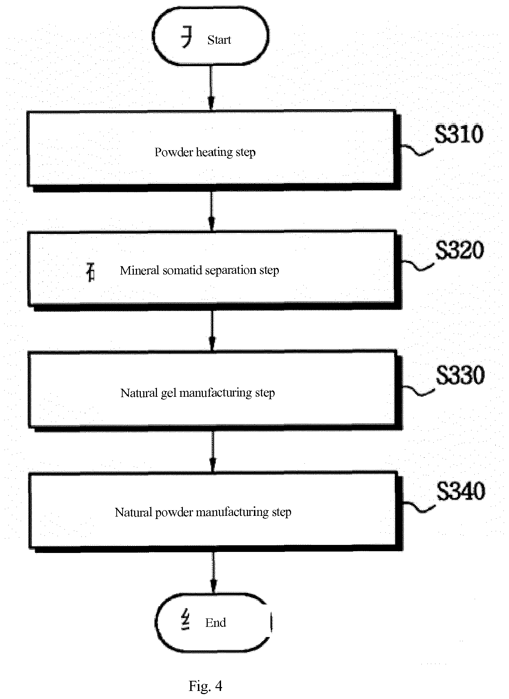

Fig. 4 is a flow diagram of the process of manufacturing oxidation-resistant natural gel and natural powder using the mineral ceramic powder produced through the process shown in Fig. 3 according to an embodiment of the present invention.

As shown in the figure, only mineral somatid is separated from the mineral ceramic powder separated through the above-mentioned steps S210∼S250, and the separated mineral somatid is manufactured into natural gel in a gel form, wherein, the mineral somatid solely consists of microbes that will not die but still survive even they are heated at high temperature of 1,000°C for 10h. Such mineral somatid is separated from the mineral ceramic powder through a powder heating step S310 and a mineral somatid separation step S320.

In other words, it means that the mineral somatid described in the present invention still survive even after the mineral ceramic powder is heated at high temperature of 1,000°C for 10h.

Moreover, the mineral somatid separated from the above-mentioned steps S310 and S320 is manufactured into natural gel and natural powder through a natural gel manufacturing step S330 and a mineral somatid natural powder manufacturing step S340.

Here, for the so-called mineral somatid, Gastong Nesang defined somatid in living bodies as tiny concrement of energy and an intermediate being between objects and life bodies. Based on that definition, in the sense of quantum energy living body, the somatid that improves vitality and provides energy for healthy growth in mineral somatid that exists in the natural world is named as mineral somatid.

The powder heating step S310 further heat the mineral somatid extracted through the procedure shown in Fig. 3 at 100∼1,000°C for 10h, to separate mineral somatid.

The mineral somatid is reheated at a high temperature, so as to confirm the result that the somatid will not die even at high temperature, as mentioned in the reports from BechamP and Gastong Nesang.

In the mineral somatid separation step S320, mineral somatid is separated from the mineral ceramic powder heated through the above-mentioned step S310. Specifically, the heated mineral ceramic powder is mixed with water or distilled water or deionized water at a certain ratio into a mixture while the mixture is stirred, and then the mixture is treated by centrifugal separation in a centrifugal separator to form a layer separation phenomenon; thus, mineral somatid is separated from the mineral ceramic powder.

In such a mineral somatid separation step S320, the mineral ceramic powder is mixed with water or distilled water or deionized water, the mixture is loaded into an stirring machine and stirred by conventional stirring or magnetic stirring for 24h, rotated in the stirring machine and a centrifugal separator for 1∼24h, and then precipitated for 3∼72h, so that layer separation happens in the mixture of mineral ceramic powder and water or distilled water or deionized water, and a layer separation phenomenon of the mineral ceramic powder is realized.

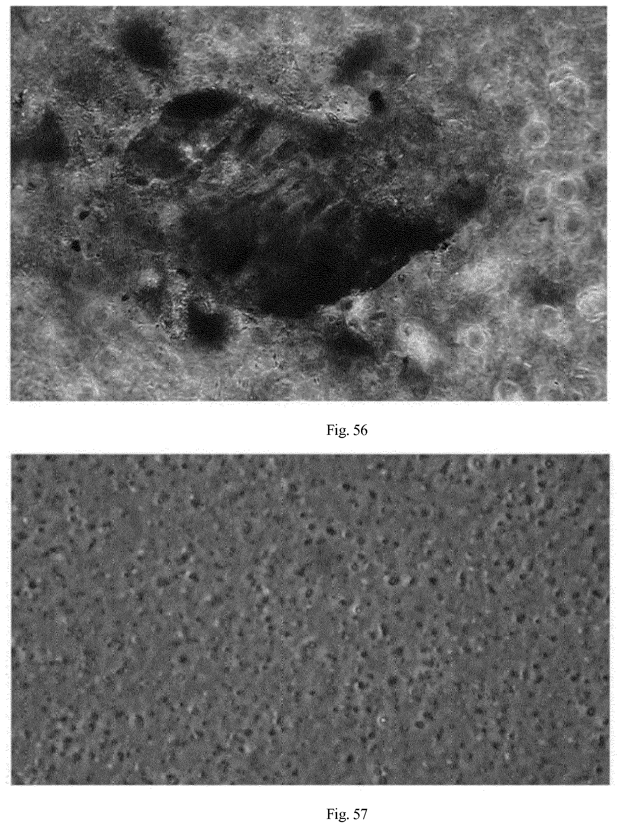

Moreover, after the precipitation is finished, the water or distilled water or deionized water is located in a top layer portion, the ceramic powder that fully contains components including SiO₂, Al₂O₃, Fe₂O₃, MgO₃, and K, etc., i.e., the mineral ceramic powder is located in a bottom layer portion, and a middle layer portion between the water or distilled water or deionized water and the mineral ceramic powder consists of a suspension and turbid liquid, in which the high-concentration mineral somatid exists. In the present invention, as shown in Fig. 57, the separation is implemented by only collecting the middle layer portion that consists of the suspension and turbid liquid.

In addition, in the content in which layer separation has happened through the mineral somatid separation step S320, the top layer portion is observed with an optical microscope, it is found that a small quantity of somatid exists in the top layer portion, which is different from the observation result of the mineral ceramic powder after the ripening and drying are finished.

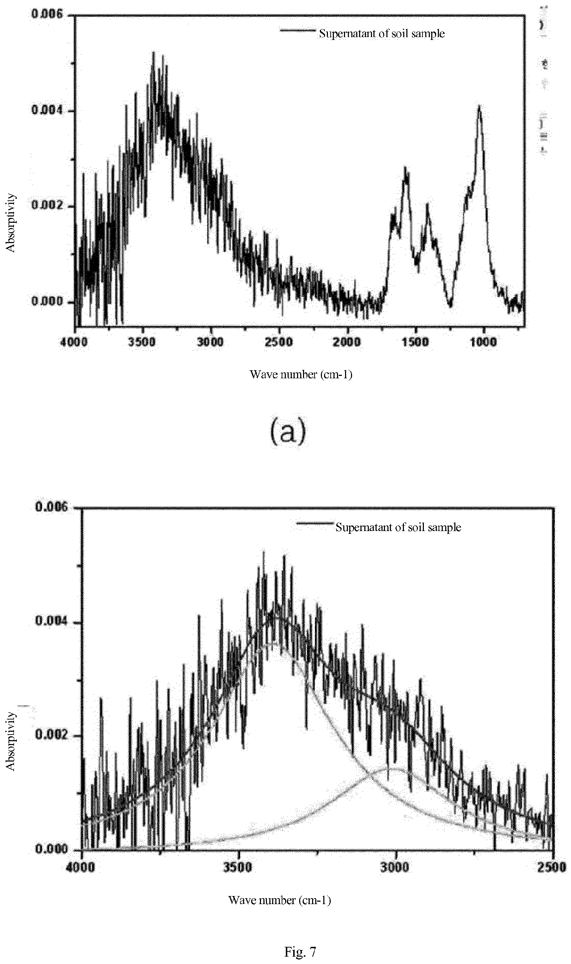

In other words, for the extracted top layer portion, as shown in Fig. 7(a), the result of FT-IR analysis shows silicon dioxide and alumina peaks; however, as shown in Fig. 7(b), the result of data fitting analysis indicates that the peaks belong to the C-H stretching vibration band, which means the existence of carbon-based organic compounds.

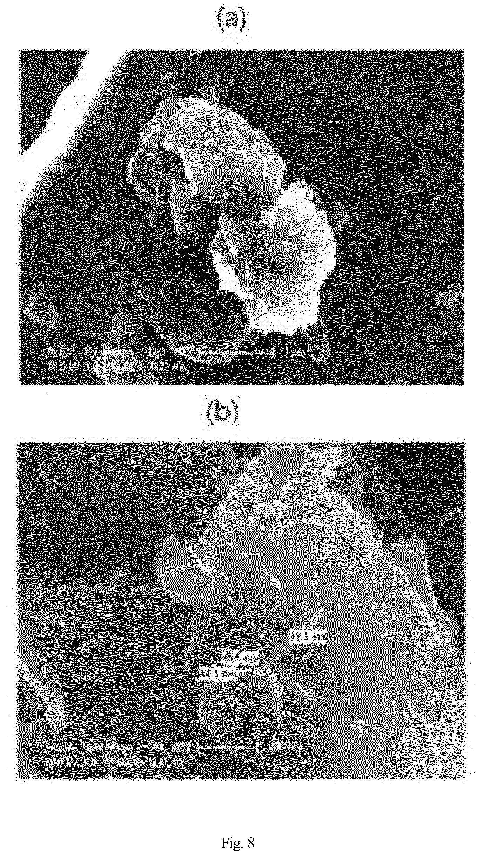

Moreover, when the top layer portion is observed by SEM after it is dried, a result similar to that shown in Fig. 8 is derived.

From Figs. 8(a) and 8(b), it is confirmed that spore morphologies in a roughly circular shape exist on the surface of the mineral ceramic powder.

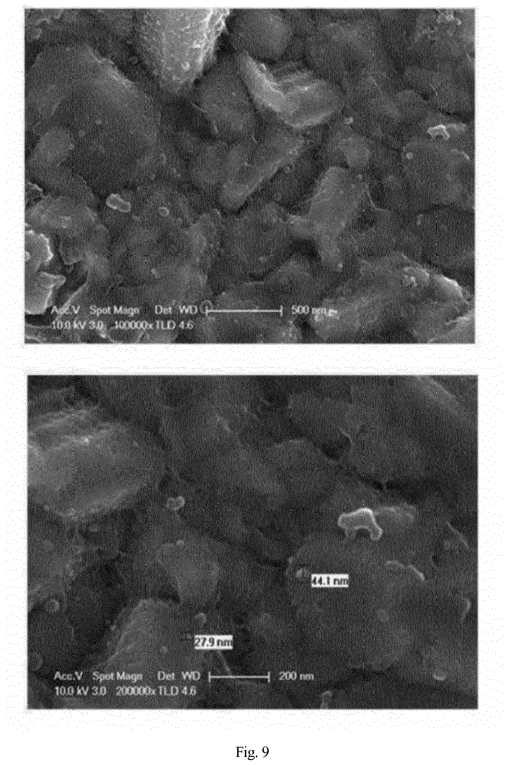

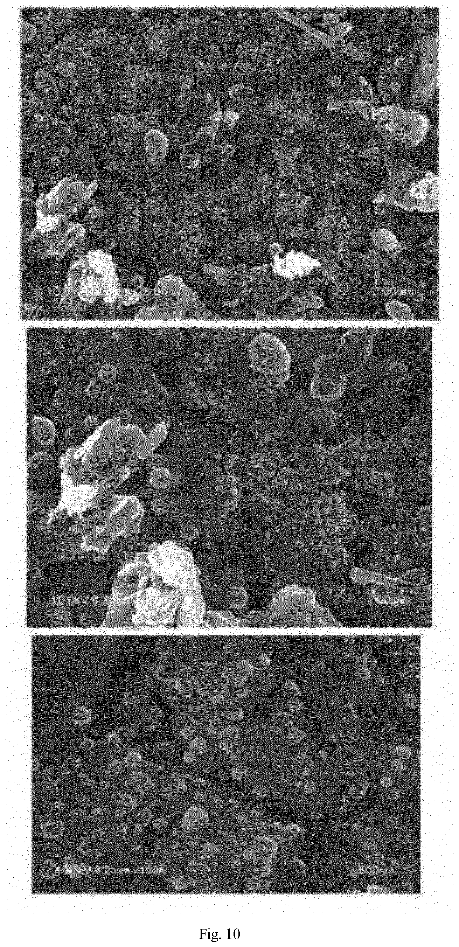

Moreover, after the centrifugal separation, mineral ceramic powder is mainly observed in the powder in the bottom layer portion. In addition, as shown in Figs. 9 and 10, a mixture consisting of a small quantity of mineral ceramic powder and a large quantity of mineral somatid is observed in the middle layer portion, and circular spore morphologies are formed on the surface of the mineral somatid.

Moreover, in the natural gel manufacturing step S330, the middle layer portion separated in the above-mentioned mineral somatid separation step S320 is heated at a specified temperature so as to evaporate water or distilled water or deionized water and extract the residual pure mineral somatid, and then the extracted pure mineral somatid is manufactured into natural gel in a gel form.

At this point, in the natural gel manufacturing step S330, the middle layer portion is heated at 100°C or a higher temperature so as to evaporate water or distilled water or deionized water, and thereby extract the mineral somatid in gel state. In other words, the natural gel solely consists of pure mineral somatid.

In the natural gel manufacturing step, the suspension and turbid liquid in the middle layer portion is loaded into a drier and heated at 100∼600°C for 1∼10h for drying, so as to evaporate the water, distilled water, or deionized water contained in the suspension and turbid liquid, and then the concentrated mineral somatid is extracted to form natural gel in a gel form.

Moreover, the natural gel manufactured through the above-mentioned step S330 contains 40∼80wt% SiO₂, 10∼35wt% Al₂O₃, 5∼10wt% Fe₂O₃, 3∼7wt% MgO₃, and 2∼8wt% K alone, or all of them respectively; then, the obtained natural gel may be mixed with a composition of food raw material, pharmaceutical raw material, anti-cancer therapeutic agent, injection and immune enhancer at a certain ratio for use.

Moreover, in the mineral somatid natural powder manufacturing step S340, the natural gel manufactured through the step S330 is treated by secondary heat treatment, and manufactured into mineral somatid natural powder through twice drying and solid content milling process.

In the mineral somatid natural powder manufacturing step S340, in order to exclude the possibility of contamination of the multi-functional natural gel extracted through the step S330, a secondary heat treatment procedure is executed to extract natural powder. In the secondary heat treatment procedure, high pressure sterilization is executed at 121°C for 15min. in an autoclave.

In other words, in the mineral somatid natural powder manufacturing step in the present invention, the multi-functional natural gel manufactured through the step S330 is dried at temperature of 100∼800°C for 2∼20h, and treated through a secondary heat treatment procedure for sterilization in an autoclave, so as to produce a solid content for manufacturing pure mineral somatid natural powder; and then the solid content is milled to produce natural powder.

Moreover, in the mineral somatid natural powder manufacturing step S340, the solid content treated through the above-mentioned secondary heat treatment procedure is stored in a drier and dried at temperature of 100∼800°C for 2∼20h, so as to fully remove the moisture content contained in the natural gel and obtain a highly concentrated solid content, i.e., the step is a solid content manufacturing step.

Then, the solid content is milled with a mill to particles in about 2nm particle size, and thereby manufactured into natural powder.

The natural gel and solid content manufactured with the method described above are the forms before the natural powder is manufactured, and contain 10∼90wt% water; especially, the solid content is 11wt% relative to water. The natural gel and solid content can be used as an additive for ointments for treating specific responses, wounds and bedsores, burnt, and skin diseases, as well as an additive for medical, food, and pharmaceutical products.

In addition, the obtained mineral somatid natural powder can be added into food additives, immune enhancers, anti-cancer drugs, anti-alcoholic drugs, blood pressure adjustment drugs, anti-diabetic drugs, and skin recovering drugs, etc., and can be used as a raw material for coating agents for IT electronics and a variety of industrial materials.

### Experiment 1

The mineral somatid natural powder manufactured through the above-mentioned steps is used as strain, and thereby an experiment of culturing mineral somatid is carried out.

The culturing result shows that the mineral somatid moves actively just like it behaves before the heat treatment, and forms very thin substance similar to a membrane.

To confirm the result, the cultured product is placed in a solution, held at room temperature for 7 days, and then observed. The solution is a water solution consisting of 10wt% syrup and 90wt% water or distilled water.

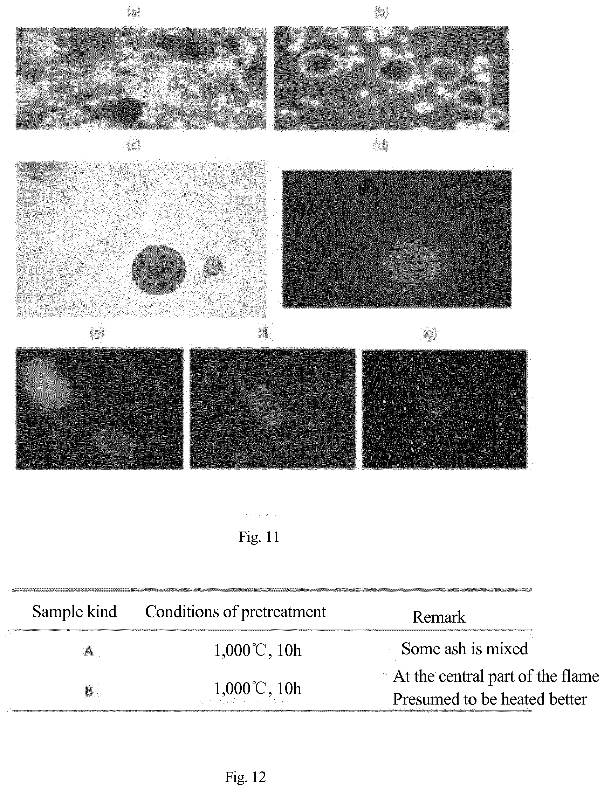

The experimental result is as follows: in the cultured product of mineral somatid treated through heat treatment, as shown in Fig. 11(a), after heat treatment is carried out through the step S310, no special phenomenon is observed; however, after 1 day, as shown in Fig. 11(b), spheres in a spherical shape begin to emerge in a large quantity, and the observation result of the spheres indicates that a clear membrane exists around the outline of each sphere, as shown in Figs. 11(c) and 11(d).

Moreover, based on the result of dyeing observation, as shown in Fig. 11(e), it is confirmed that a phospholipid layer exists; in addition, as shown in Figs. 11(f) and 11(g), DNA is perceived.

This means a self-organization and nucleic acid and cell formation process has happened owing to the existence of the mineral somatid. Compared with the development of mineral somatid in cell morphology, it is comprehended that the mineral somatid maintains its original state and assembles the peripheral nutrient substances and thereby produces cytoplasm.

### Experiment 2

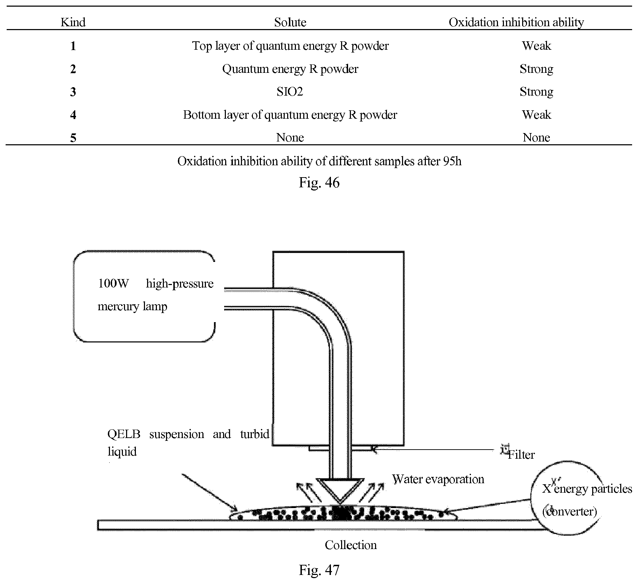

In addition, as shown in Fig. 47, an experiment on the response of the multi-functional natural gel or natural powder manufactured through the above-mentioned steps under irradiation of UV and visible light is carried out.

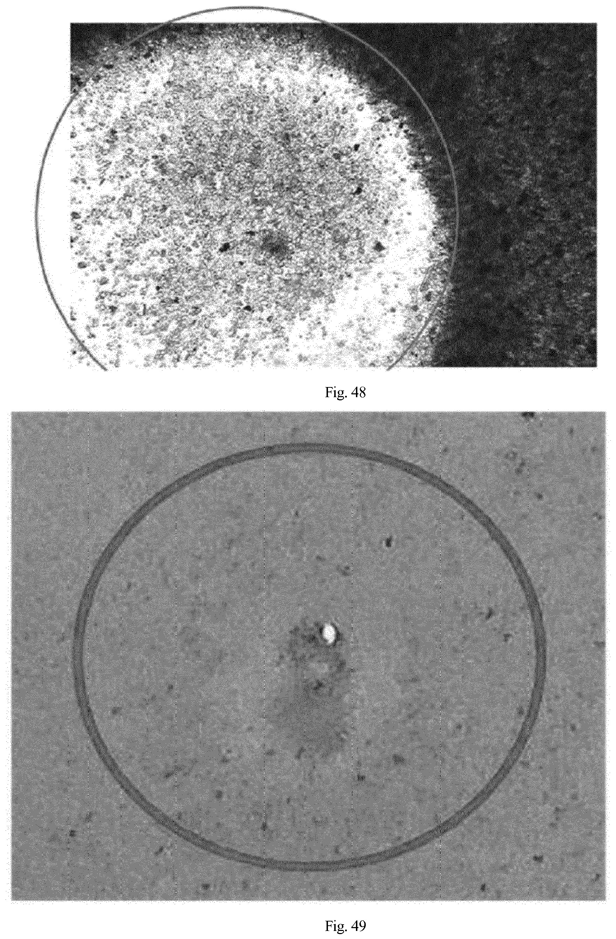

The experimental result is as follows: under the irradiation of ultraviolet light, the moisture content is vaporized instantly and dehydration happens; at the same time, the activity of the mineral somatid is improved; moreover, as shown in Figs. 48 and 49, it is confirmed that electromagnetic wave responsive energy is generated.

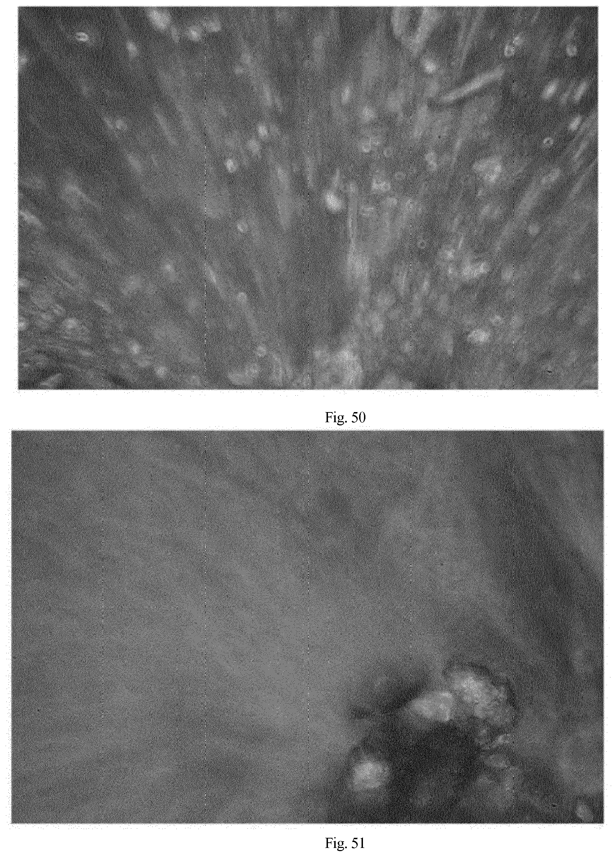

In other words, as shown in Figs. 50-52, under irradiation of ultraviolet light and visible light, it is confirmed that the water contained in the natural gel and solid content rotates and emits energy in a doughnut form towards the center of the light under the energy emitted from the mineral somatid, and thereby specified power is generated.

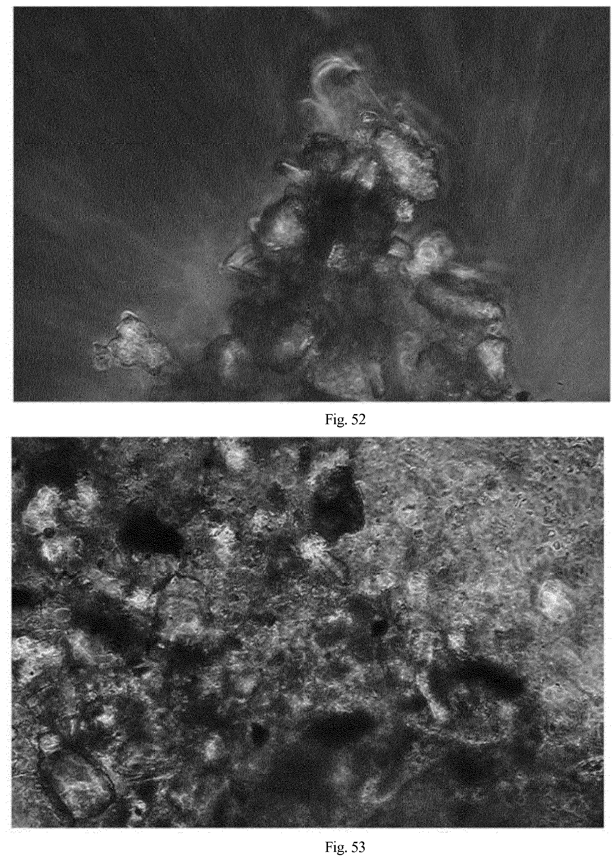

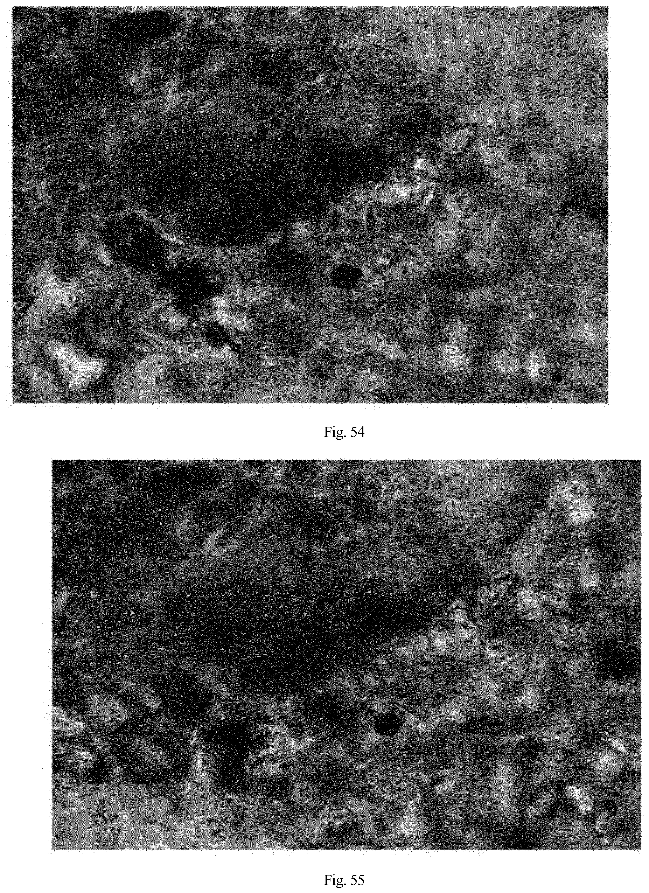

Furthermore, as shown in Figs. 53-56, under irradiation of ultraviolet light and visible light, it is confirmed that the edible oil contained in the natural powder rotates and emits energy in a doughnut form towards the center of the light under the energy emitted from the mineral somatid, and thereby specified power is generated.

Here, Fig. 48 shows the experimental result of the change of mineral somatid under UV irradiation, wherein, the red circle represents the scope of UV irradiation, and reflects the change incurred by the energy increase phenomenon of the mineral somatid that exists within the scope of UV irradiation. Moreover, Fig. 49 shows the result of UV irradiation, and reflects water vaporization on the circumference of the scope of UV irradiation.

Under irradiation of ultraviolet light and visible light, the multi-functional natural gel or natural powder described in the present invention react with the light, and thereby the energy emitted from the mineral somatid is increased. The effect of energy increase can be utilized in a variety of industrial raw materials.

### Experiment 3

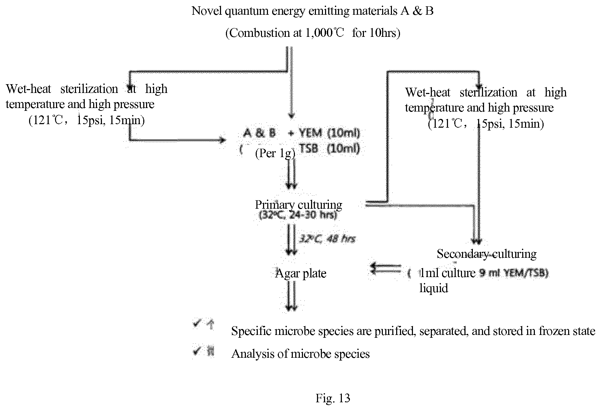

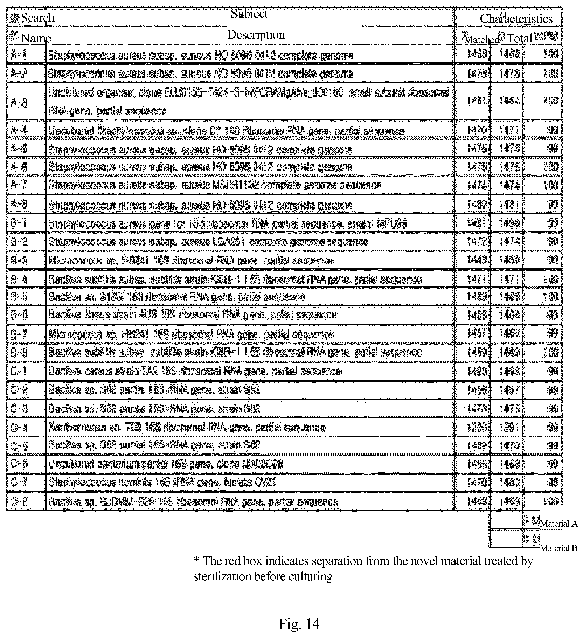

Hereunder the possibility of microbial fusion in the mineral powder of mineral somatid after pre-treatment at a super-high temperature will be investigated. Here, the anti-microbial ability against various pathogenic bacteria is experimented qualitatively and quantitatively, besides the potential microbial species are explored and identified.
1. In order to explore the microbial species contained in the mineral power and analyze their functions, as shown in Fig. 12, two samples (samples A and B) are pretreated and then experimented.
   Then, the microbes contained in the samples A and B that have been pretreated are cultured, and are experimented according to the strategic pattern for microbe species exploration, separation, and identification shown in Fig. 13.
2. Minimum nutrient medium and complex nutrient medium, which are extensively used, are used as culture medium for investigating the possibility of microbial fusion in the mineral powder that contains mineral somatid and multiple microbial species.
   As the minimum nutrient medium, yeast extract minimal medium (YEM) is produced in a formulation of 3.5g of Na₂HPO₄·12H₂O, 1.0g of K₂HPO₄, 0.03g of MgSO₄·7H₂O, 0.5g of NH4C1, 4.0g of yeast extract, and 1L of D.W.upto, and then is sterilized for use.
   As the complex nutrient medium, trypsinase soy broth (TSB, from BD company) is used; the solid culture medium are produced by adding 1.5% (w/v) bacto-agar in the formulations of YEM and TSB.
   The microbe culturing is to hold at 32°C temperature for 24∼72h, and aerobic culturing and anaerobic culturing are tried; in the case of liquid aerobic culturing, microbe culturing is tried in a shaking incubator under 250rpm condition; in the case of anaerobic culturing, an automatic anaerobic and microaerobic bacteria culturing system (Anoxomat Mark II System) is used for anaerobic culturing.
3. As shown in Fig. 14, to identify the microbial species, after the cell shapes of all separated microbes are observed under a microscope, the genomic DNAs are separated and purified through a CTAB process, and are analyzed with a BLAST program (http://blast.ncbi.nlm.nih.gov/Blast.cgi).
4. The experimental result is as follows: altogether 53 species of aeromicrobes that are harmless to living bodies are purified and separated from the samples A and B. The results of molecular genetic identification of 24 species among the aeromicrobes show: among the 24 species of aeromicrobes, 13 species come from the mineral powder sample A, 11 species come from the sample B, wherein, 3 species are separated from samples A and B that are treated by wet-heat sterilization at high temperature and high pressure before culturing, and 21 species are separated from samples A and B that are not treated by sterilization.

In addition, tiny microbes in about 1µm size exist in the microbes separated from the samples A and B that are not sterilized. It is worthy to note that a substance in membrane morphology is also formed on the bottom surface of the Petri dish that is used for the culturing, which is similar to the experimental result in the step S340.

Especially, to ascertain whether there is any fault in the experiment, the cultured microbes are separated independently, sterilized in an autoclave at 121°C temperature for 15min., and then are cultured again, but the culturing is failed; however, if microbes in the ordinary environment, which are not cultured, are placed together with the mineral somatid natural powder into the culture medium, sterilized at 121°C temperature for 15min., and then cultured again, the culturing is successful.

It is seen from the above result: the mineral somatid has an effect of protecting and save ordinary microbes in an extreme environment. At the same time, the result of observation of proliferation of microbes after the microbes are separated and sterilized under pressure without adding the mineral somatid natural powder shows that all of the microbes have been killed and no microbe proliferation has happened.

That indicates the mineral somatid natural powder can provide a shelter for ordinary microbes against high temperature, or the mineral somatid natural powder helps ordinary microbes to conquer high temperature by virtue of its energy effect.

### Experiment 4

Hereunder an experiment for understanding the functions and effects of the above-mentioned mineral somatid will be implemented. Especially, in order to qualitatively and quantitatively analyze the anti-microbial ability of the mineral powder (i.e., mineral somatid) that is treated by wet-heat sterilization at high temperature and high pressure before the culturing, the following experiment is carried out with a disc diffusion method and broth dilution method, according to the standard methods specified in the guide book issued by Clinical Laboratory Standards Institute (CLSI).

### 1. Disc diffusion method

### Conditions of experiment

The target strain for anti-microbial ability test is cultured in Mueller Hinton broth (MHB, from BD company) at 35°C for 2-6h, the concentration of the microbes is adjusted to McFarland No. 0.5, and then the microbes are inoculated with a sterilized cotton swab to Mueller Hinton Agar (MHA). Here, the microbes are inoculated within 15min. after they are diluted, the Petri dish is rotated by 60° and the microbes are spread for 3 times consecutively on the Petri dish during the inoculation.

After the spreading, the culture medium is dried for 3∼5min. at normal temperature, and 50% (w/v) suspension and turbid liquid of mineral somatid is inoculated with a inoculating loop within 15min. The 50% (w/v) suspension and turbid liquid of mineral somatid is provided by adding mineral somatid into 0.15M sterilized NaCl saline solution at 1:1 ratio. After culturing at 35°C for 16∼18h, the size of the inhibition zone is observed, and thereby the anti-microbial effect is measured.

### Result of experiment

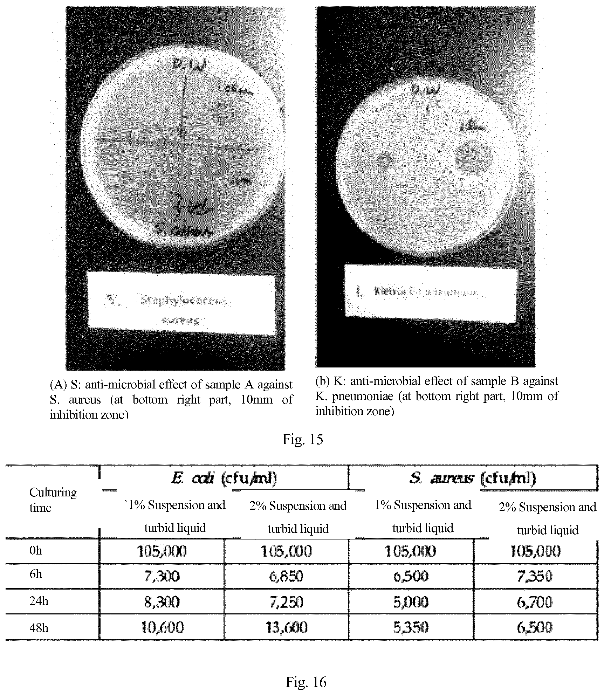

As shown in Fig. 15, the investigation result of anti-microbial effect with the disc diffusion indicates that an inhibition zone (indicated by a red circle) is formed in S. aureus and K. pneumoniae. However, no such inhibition zone is found in other bacteria species except the above species. Though the cause is unknown, based on such a result, it is confirmed that the mineral somatid exerts a stronger anti-microbial effect in S. aureus and K. pneumoniae.

### 2. Broth dilution method

### Conditions of experiment

After a single colony is activated through sub-culturing, it is inoculated into 3ml of bactericidal saline solution; thus, the turbidity is adjusted to McFarland standard turbidity 0.5. After the adjustment, 100µl of adjusted microbial solution is diluted in a flask that contains 100ml of bactericidal saline solution, and thereby the concentration of microbes is adjusted to 1x10⁵ CFU/ml, and suspension and turbid fluid of mineral somatid is added so that the ultimate concentration is 1% and 2% respectively. Then, the solution is kept at room temperature for reaction. 1ml of microbial solution is taken at 0h, 6h, 24, 48h, and 72h respectively, and the microbial counts are measured and compared.

### Result of experiment

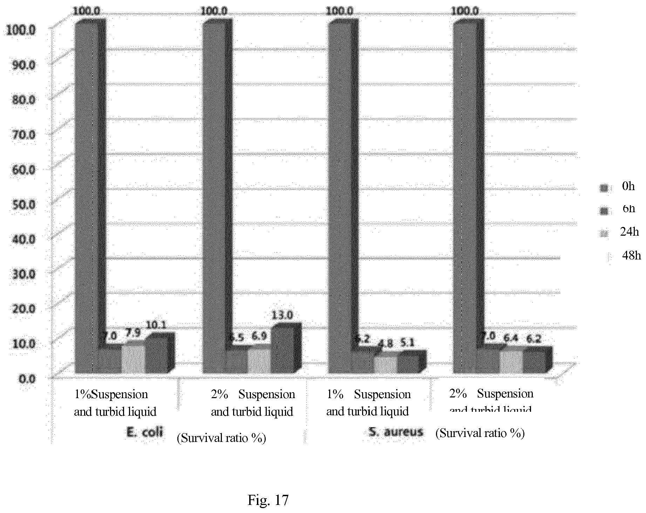

The results of quantitative analysis of the anti-microbial effect to E.coli ATCC 25922 and S.aureus ATCC 29213 carried out with the broth dilution method are shown in Figs. 16 and 17. It is confirmed that an anti-microbial effect begins to appear quickly starting from 6h after the E.coli and S. aureus are cultured with 1-2% (w/v) suspension and turbid liquid of mineral somatid. It is confirmed that the survival rate of the microbes after 6h is irrelevant to the concentration of the suspension and turbid liquid, and is decreased by 93% approximately.

### Experiment 5

VOC and ammonia removal experiments of mineral somatid are implemented, in order to understand the functions and effects of the mineral somatid extracted through the above-mentioned steps.

### VOC removal experiment

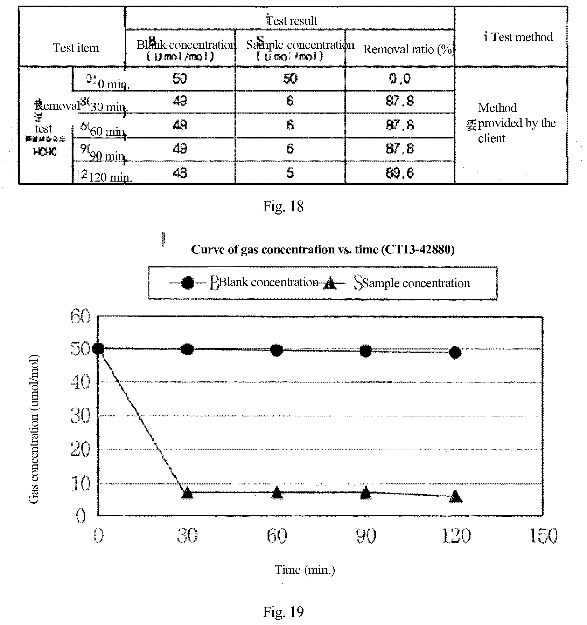

As shown in Figs. 18 and 19, in the VOC removal experiment of the mineral somatid in the present invention, 87.8% removal ratio is attained after at least 30min., and 89.6% removal ratio is attained after 120min.

### Ammonia removal experiment

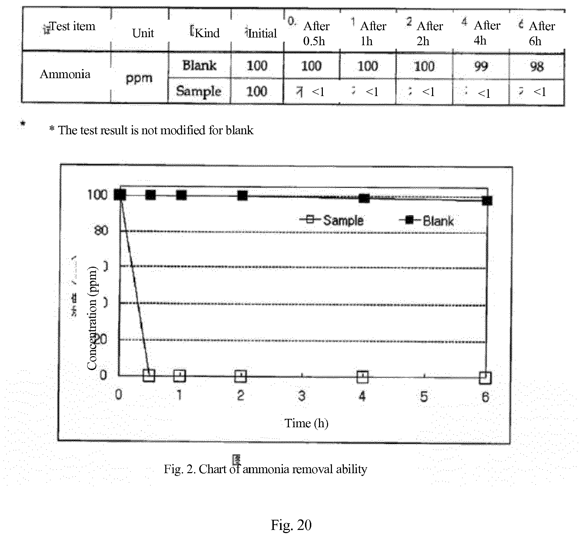

As shown in Fig. 20, under the condition of 100ppm initial concentration, it is confirmed that a great change of decreasing to 1ppm or a lower value happens after 0.5h.

Thus, it is evident that the mineral somatid in the present invention attains a good VOC and ammonia removal effect.

### Experiment 6

### -- Experiment on electromagnet wave absorption and shielding capability

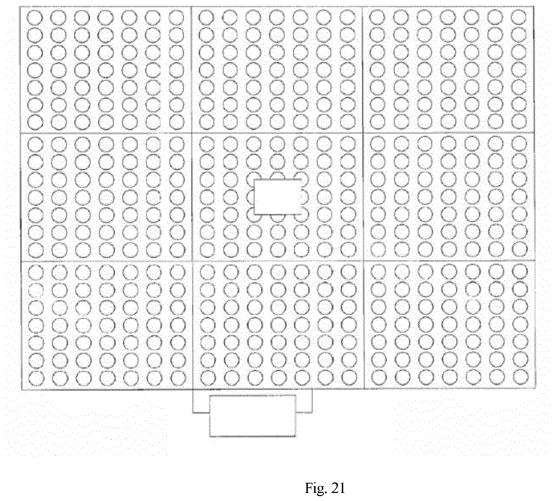

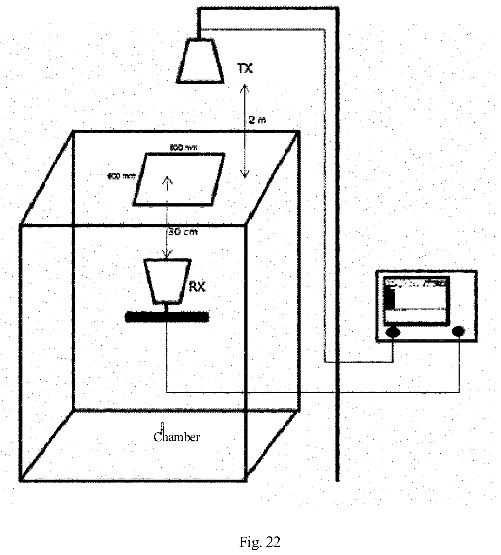

As shown in Figs. 21 and 22, in the experiment on electromagnet wave absorption and shielding capability, the electromagnet wave absorption capability is verified with a simple instrument with a time domain reflectivity test method for an electromagnetic wave absorber, wherein, the electromagnetic wave absorber is produced with carbon, rosin and loess into a panel shape. In addition, the electromagnetic wave shielding capability is verified with a shielding measurement instrument with the method specified in IEEE Std 299 & MIL-STD-188-125.

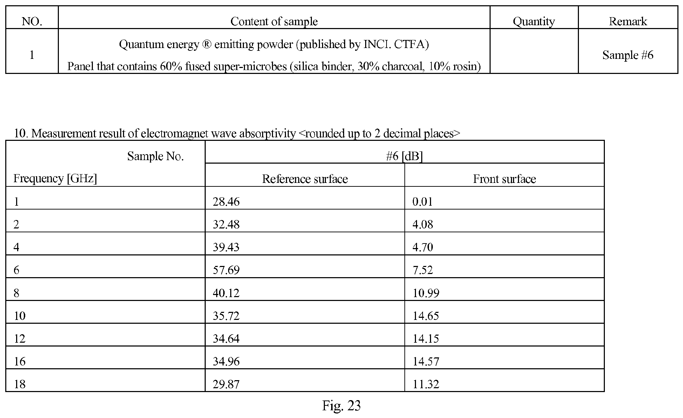

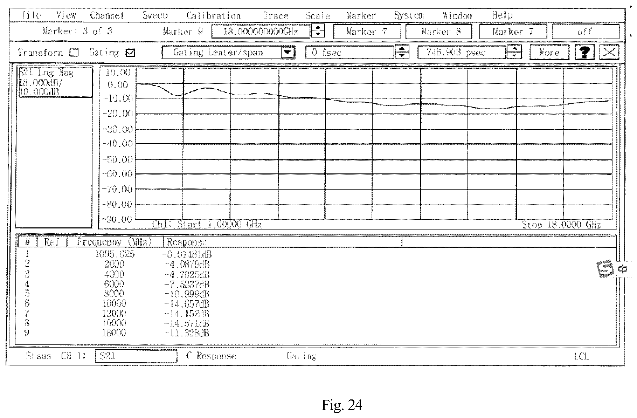

In the experiment on electromagnet wave absorption and shielding capability, a panel made with 60% mineral somatid in the present invention, 30% charcoal, and 10% rosin is used for testing the electromagnet wave absorption capability. The result of experiment of the electromagnet wave absorptivity is shown in Figs. 23 and 24.

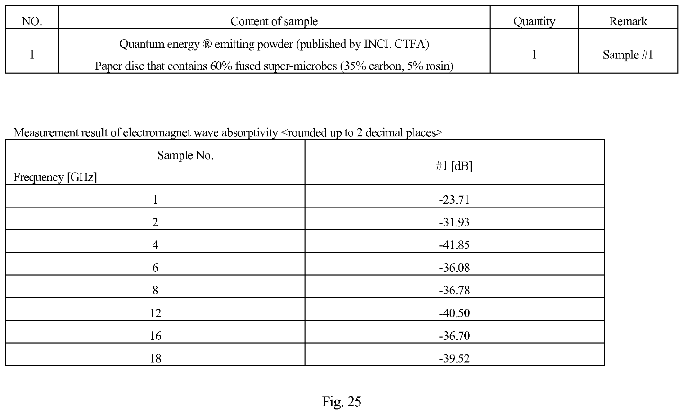

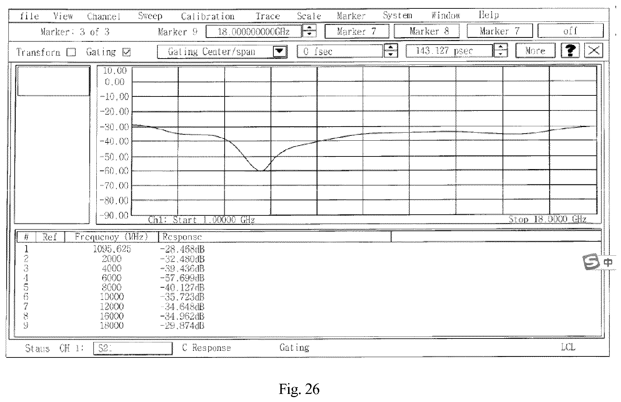

In addition, a panel made with 60% mineral somatid, 35% carbon, and 5% rosin is used for testing electromagnetic wave shielding capacity. The result of experiment of the electromagnetic wave shielding efficiency is shown in the charts in Figs. 25 and 26.

### Experiment 7

### -- Experiment on heat generation performance of mineral somatid

The mineral somatid in the present invention is tested with the experimental method for "EL610 ice removal agent", to confirm whether the ice can be thawed at -12°C and -5°C, so as to test the heat generation performance of the mineral somatid.

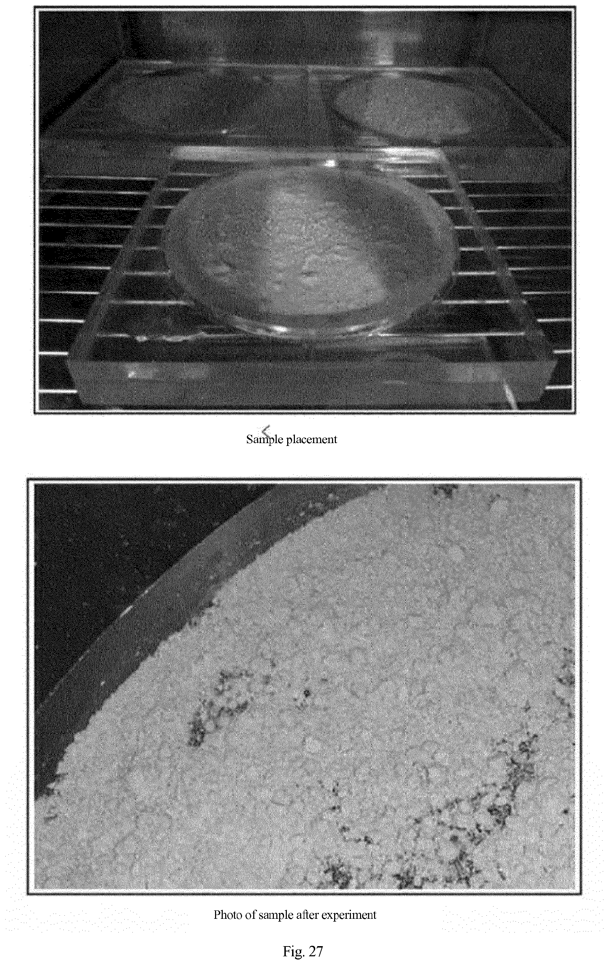

The experimental result indicates: as shown in Fig. 27, the sample in the experiment fails to thaw the ice completely under condition of -12°C; however, after the experiment, it is found that the particles of the sample agglomerate in a spherical shape.

### Experiment 8

### -- Experiment on current charge effect of mineral somatid

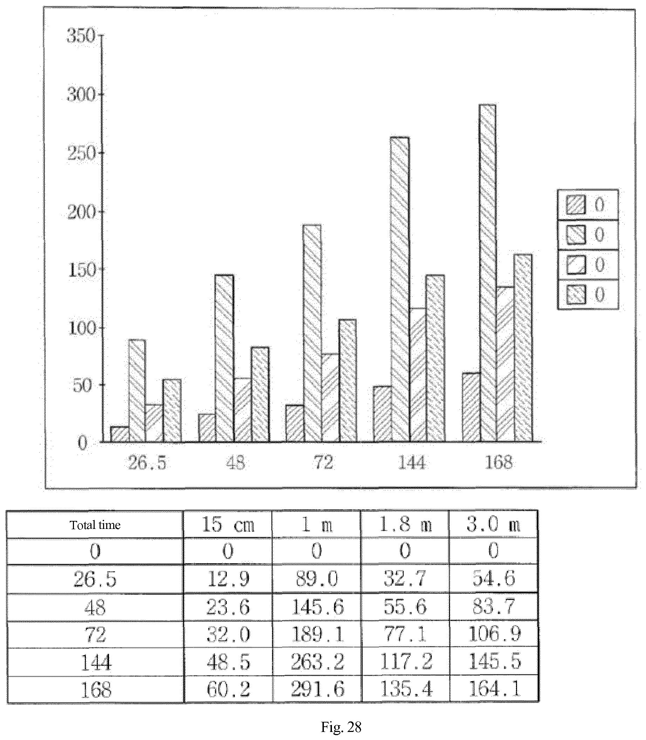

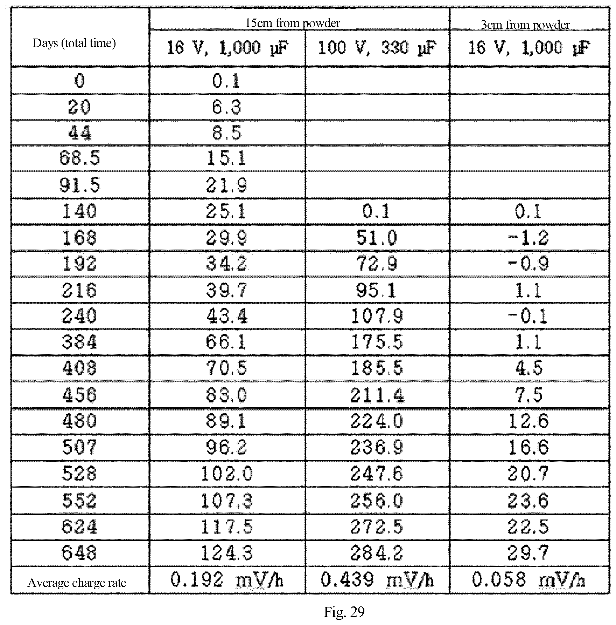

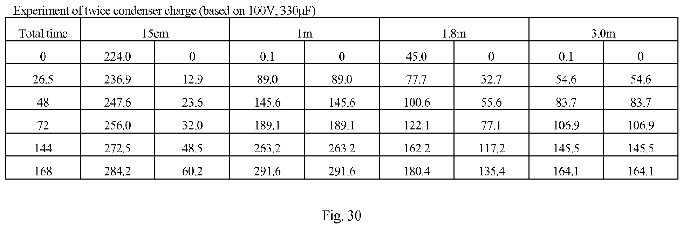

An experiment on the current charge effect of mineral somatid is implemented. As shown in Figs. 28-30, an electrical condenser that discharges at 0.1mV or lower voltage is self-charged through mineral somatid.

The experimental result is as follows: the condenser placed at 15cm (purple), 1m (amaranth), 1.8m (beige) and 3.0m (light blue) from the mineral somatid in the present invention is self-charged at a current of 12.9mV (min.)∼164.1mV (max.) within 26.5∼168h.

### Experiment 9

### -- Measurement of zeta potential of mineral somatid

1. Sample information
   (1) Name of sample: Quantum nanometer powder (hereinafter referred to as mineral somatid)
2. Analytical method
2-1. Zeta potential
   (1) Measuring instrument: zeta potential & particle-size analyzer ELSZ-2 from Photal OTSUKA ELECTRONICS company.
   (3) Principle: Electrophoretic light scattering (Laser Doppler)
   (4) Light source: Laser diode
   (5) Measurement range
      - Concentration range: 0.001 % ∼ 40 %*1
      * - Zeta potential: -200 ∼ 200 mV
      - Electric mobility: -10 ∼ 10 µm·cm/sec·V
2-1-1. Conditions of dispersion solvent
   (1) Solvent: H₂O
   (2) Temperature: 25 °C
   (3) Refractive Index: 1.332 8
   (4) Viscosity: 0.887 8 cp
   (5) Dielectric constant: 78.3
3. Result of analysis
3-1 Measurement of dispersion safety

**[Table 1] Measurement Result of Zeta Potential (Dispersion Safety)**

| Name of Sample | Unit | Result of Analysis |
|---|---|---|
| Koptri-1330182-1 | mV | -37.74 |

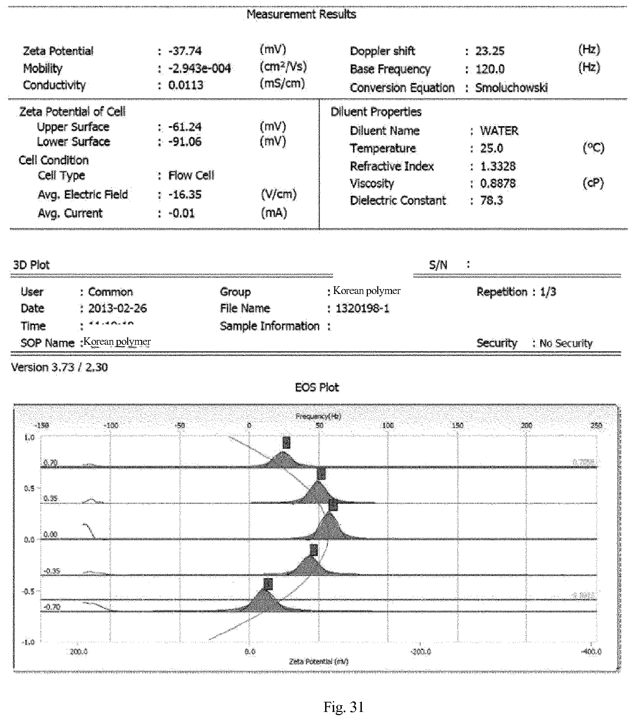

As described above, for the experiment of measurement of zeta potential of mineral somatid, as shown in Fig. 31, from the viewpoint of Gastong Nesang, who asserted that the somatid in living bodies carries negative charges, the result of the experiment on the zeta potential of mineral somatid natural powder indicates that the zeta potential in natural state is 37.7mV, which is a quite high value.

In addition, if the mineral somatid carries negative charges, the cation exchange capacity is different from that of ordinary ceramics, and thereby can be measured.

Here, the CEC value is 99.87cmol+/kg, which is also a quite high value. Furthermore, since the mineral somatid natural powder carries negative charges, it is anticipated that the reducing power of the mineral somatid is strong.

### Experiment 10

### -- Experiment on oxidation-resistant effect of mineral somatid

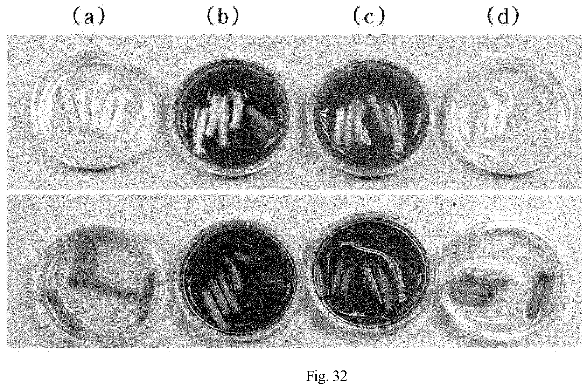

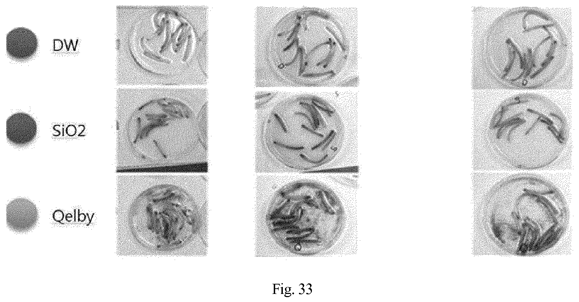

In order to confirm the oxidation-resistant effect of a solution that contains mineral somatid again, as shown in Figs. 32 and 33, an experiment on the oxidation-resistant effect of mineral somatid is made. The result is as follows: after the mineral somatid natural powder is precipitated for 1h at Ig gravity in water or distilled water in an appropriate amount, the top layer and bottom layer are separated and collected for use.

The stems of bean sprouts are cut into appropriate size and collected, and immersed in suspension and turbid liquids, and the change of the stems with time is observed for about 140h. The solutes suspend at 0.01g/ml concentration in water or distilled water, and are applied to the bean sprouts. After that, in consideration of the reduction of suspension and turbid liquid, 0.1ml of water or distilled water is supplemented once every day.

Fig. 32 shows the change after 140h. Fig. 32(a) shows water or distilled water, Figs. 32(b) and 32(c) show top layer and bottom layer of mineral somatid, Fig. 32(d) shows water mixed with silicon dioxide. In addition, 10mL of DW is used as the solvent.

As shown in the figures, the bean sprouts placed in the top layer and bottom layer (b, c) of suspension and turbid liquid of mineral somatid exhibit a better tissue necrosis and deterioration inhibition effect than the SiO₂ (d) (control group) or untreated group (a). Compared with the bean sprouts in the untreated group and SiO₂ treated group, which turn into yellow and are deteriorated, the bean sprouts placed in the suspension liquid of mineral somatid remain in the original state.

Based on the result, it is confirmed that the stems of bean sprouts placed in water or distilled water, or water with dissolved silicon dioxide are oxidized, and thereby the tissue cells are deteriorated or necrosed, and the stems become loose; in contrast, the stems of bean sprouts in water with dissolved mineral somatid remain fresh. The result is irrelevant to the position where the mineral somatid is separated and collected after centrifugal separation, i.e., the result is almost the same, regardless of whether the middle layer or top layer of the liquid is used. That means a small quantity of mineral somatid also exists in the top layer (supernatant).

### Experiment 11

the experiment 11 is carried out after the above-mentioned experiment on the oxidation-resistant effect of mineral somatid is finished, under the same conditions, the quantity of generated biophotons is measured with a photomultiplier tube.

### -- Experiment on measurement of the quantity of generated biophotons related with mineral somatid with a photomultiplier tube

All organic bodies emit tiny photons, which are referred to as biophotons. The tiny photons are generated under the biochemical actions among all cells of an organic body. It is well known that biophotons are an active research domain related with researches on oxidation resistance. Though the released quantity of biophotons is not a direct indicator for oxidation, it is related with life and death of cells and has an important influence.

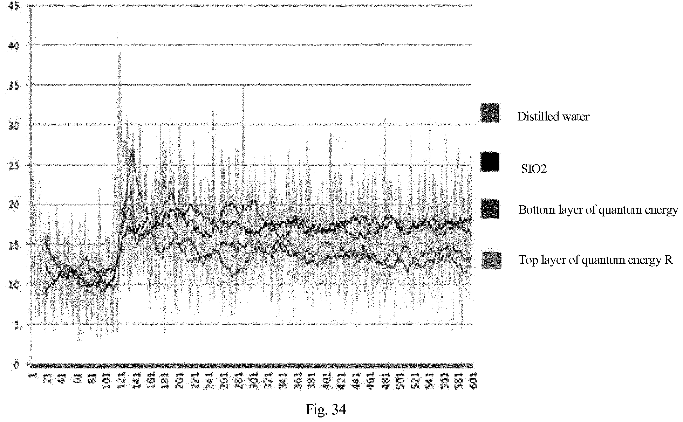

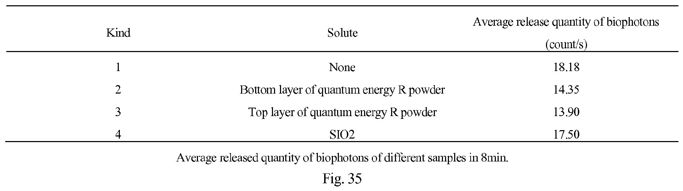

Fig. 34 shows the chart of measurement result of the quantity of generated biophotons with a photomultiplier tube. The released quantities of biophotons are similar in terms of time and type, but the average released quantities of biophotons are different. Fig. 35 shows a chart of average quantities of biophotons released from the samples in 8min.

According to the result, the quantities of biophotons released from the samples of bean sprouts immersed in water or distilled water and the samples immersed in water with dissolved SiO₂ are the highest and about the same. Thus, it is ascertained that there is no oxidation-resistant effect in the water with dissolved SiO₂. The quantities of biophotons released from the bean sprouts immersed in the top layer and bottom layer of mineral somatid are similar to each other and lower. Observed with naked eyes, the quantity of biophotons released from deteriorated bean sprouts is higher, which is in line with the phenomenon that the quantity of biophotons released from a plant is increased when the plant is damaged by herbicide that attains an oxidation effect in a prior research. Thus, it is confirmed that the mineral somatid has an oxidation inhibition effect, and the conclusion that the released quantity of biophotons is changed as the bean sprouts are deteriorated with time is verified again.

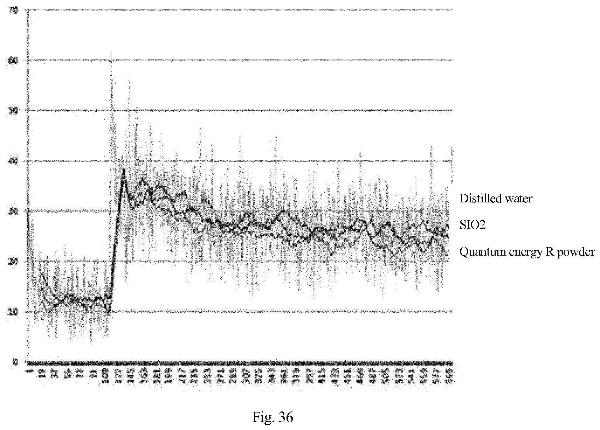

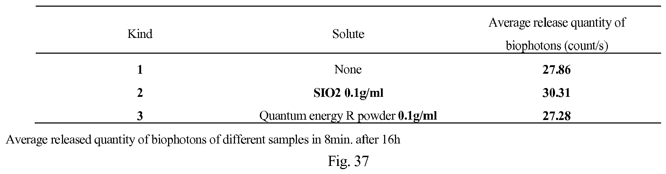

Figs. 36 and 37 show the chart and data sheet of result of re-verification of the released quantity of biophotons after holding for 16h initially.

When observing the measurement result, the fragments of bean sprouts after 16h are similar in state, and still exhibit vitality. According to the result of visual observation, it is believed that the state of the fragments of bean sprouts and the released quantity of biophotons are related with each other, and the average quantity of released biophotons in the SiO₂ group, which is slightly increased, exhibits a value similar to the measured value corresponding to the degree of deterioration observed visually.

Thus, it can be judged more clearly and definitely that SiO₂, which is a main constituent of mineral somatid, does not have deterioration inhibition ability in living bodies; accordingly, it can be confirmed that specific elements of mineral somatid have deterioration inhibition ability in wound plants.

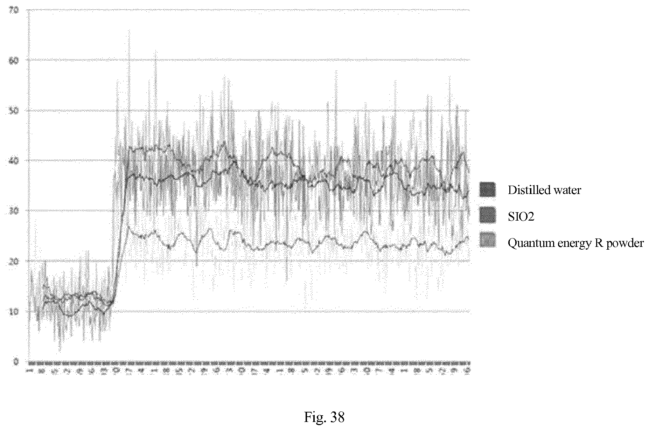

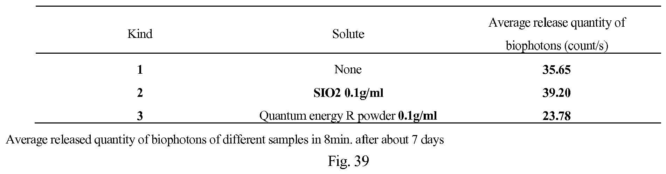

Figs. 38 and 39 show the chart and data sheet of result of re-verification of the released quantity of biophotons after holding for 7 days.

The degree of biophoton release in about 7 days is measured. It is observed that the deterioration inhibition ability of mineral somatid in the plant still exists apparently, and all the plants in the treated groups are deteriorated within 7 days, except the plants in the group treated with mineral somatid.

Later, we plan to study the mechanism when making the following experiments: The influence on additional organic bodies will be learned through experiments on animal cells by means of MTT assay and DPPH assay, etc.

In order to verify the reducing power of mineral somatid also functions in a contactless manner, a beaker that contains water is placed on fused powder of mineral somatid to measure pH and oxidation-reduction potential (ORP) in a contactless manner.

The measurement result indicates that the pH changes to a value in a slightly alkaline range, and the ORP is decreased from 50mV to 10mV and remains at that value. The pH change to a value in slightly alkaline range which means the concentration of H⁺ ions in the water is decreased, i.e., the quantity of negative charges is increased in the water.

The decrease of ORP is also in line with the increase of the quantity of negative charges in the water finally. Such a result means that the energy emitted from the mineral somatid can attains a reducing effect through the glass beaker in a contactless manner, even though the fused powder of mineral somatid is not in direct contact with the water.

### Experiment 13

### -- Experiment on oxidation inhibition efficacy of mineral somatid

### 1. Conditions of experiment

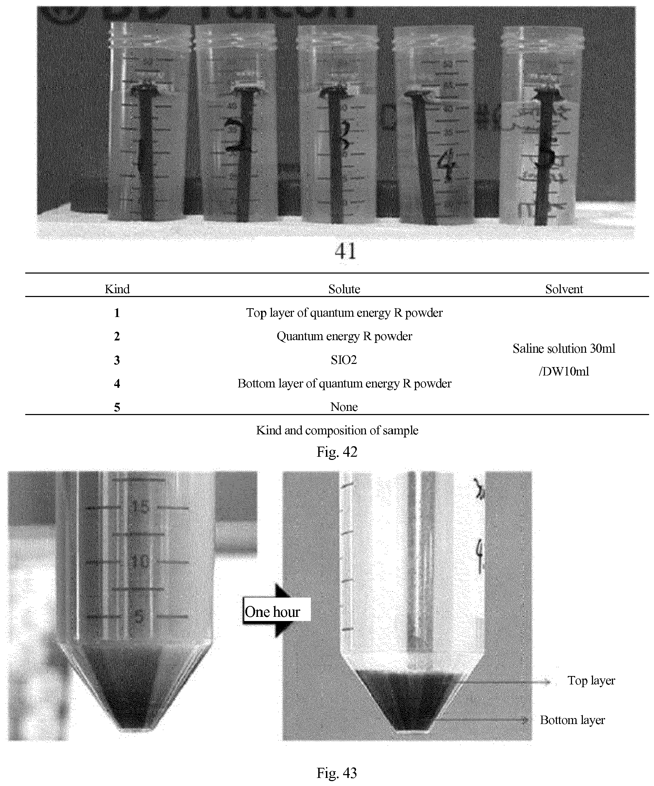

In order to test the oxidation inhibition efficacy of mineral somatid, as shown in Fig. 41, the oxidation of spikes is boosted with saline solution; SiO₂, which is a main constituent of mineral somatid, is added into the control group, so as to further confine the target of the subject of clinical effectiveness. It is confirmed that the mineral somatid is precipitated at 1g gravity and forms two layers; then, the mineral somatid is separated and extracted, and the target of oxidation inhibition effect is traced.

### 2. Method of experiment

Spikes are immersed in saline solutions at the same concentration and the same quantity, and the degrees of rust of the spikes are measured at specified times. The composition of the solution is shown in Fig. 42.

The quantities of mineral somatid and SiO₂ used in the suspension and turbid liquid of mineral somatid are fixed to 0.1g/ml respectively. In addition, as shown in Fig. 43, the mineral somatid is suspended in water or distilled water at 0.1g/ml concentration and precipitated at Ig gravity for 1h to form top layer and bottom layer; thus, the sample obtained by separation and extraction is suspended in a solvent (saline solution) again.

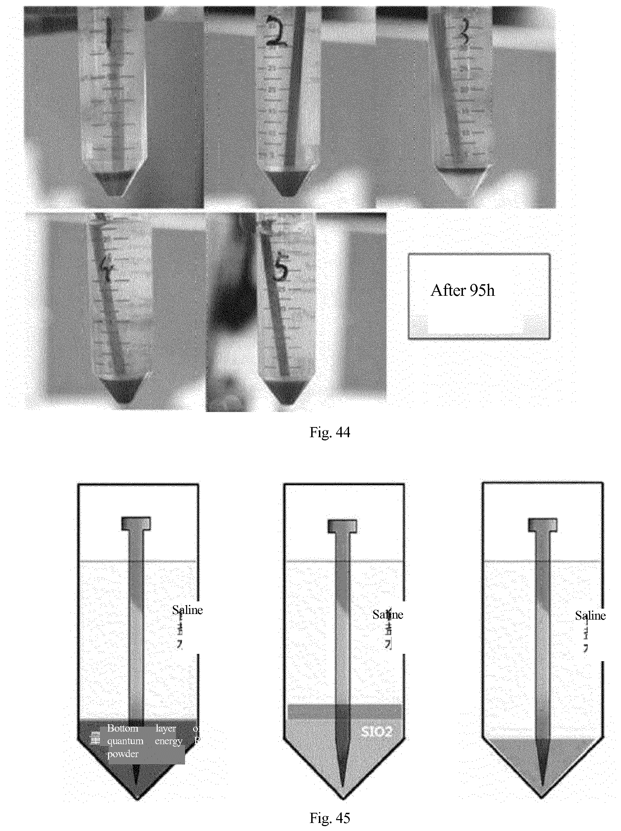

As shown in Fig. 44, after the sample is held at room temperature for about 95h, the degree of rust is measured, to compare the oxidation inhibition ability of the mineral somatid. Moreover, as shown in Fig. 45, the bottom layer of suspension liquid of mineral somatid exhibits the highest oxidation inhibition ability when compared with other control groups. The relative oxidation inhibition ability of the sample is observed with naked eyes, as shown in Fig. 46.

### 3. Result of experiment

In the experiment, the oxidation inhibition effect of the suspension and turbid liquid of mineral somatid is exhibited very clearly, and the degree can be confirmed easily even with naked eyes.

According to the analytical result, it is judged that the main body of oxidation inhibition ability in the suspension and turbid liquid of mineral somatid exists in the substance in the bottom layer separated through precipitation at 1g gravity for 1h, and SiO₂, which is a main constituent of mineral somatid, is not the cause.

### Experiment 14

### -- FT-IR, TGA, XRF, and XRD experiments of multi-functional natural gel and natural powder

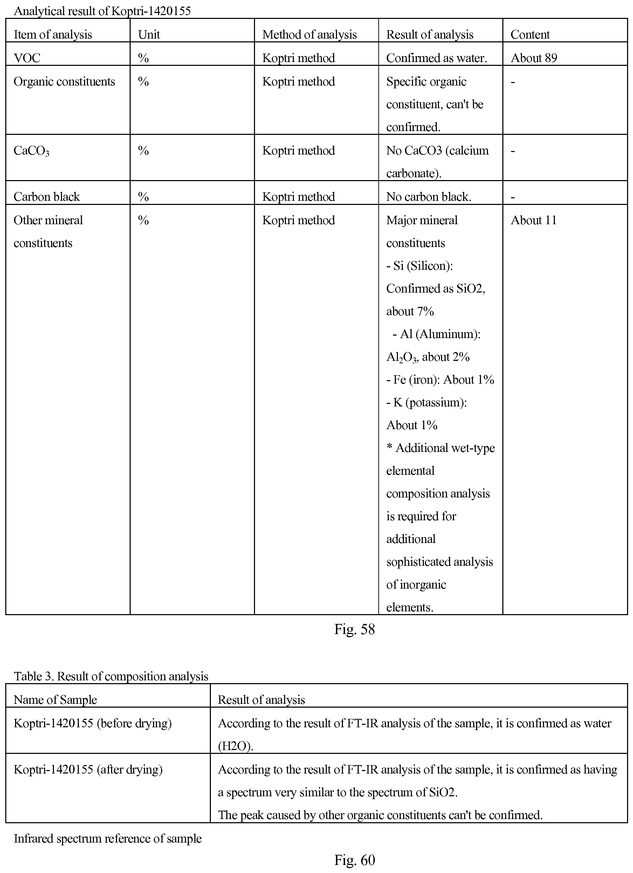

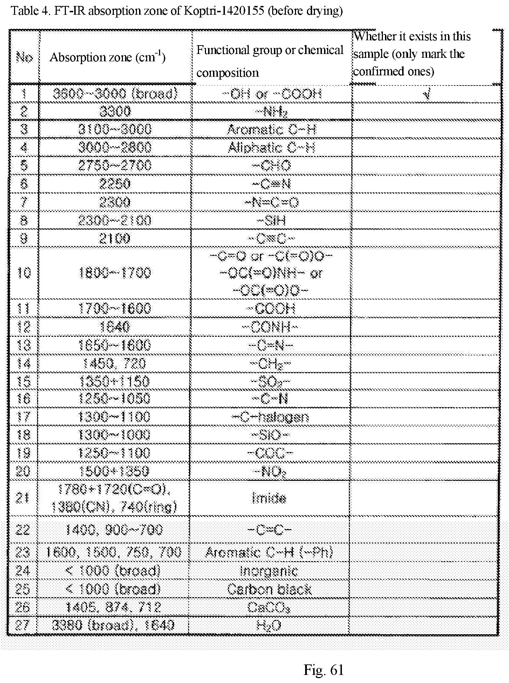

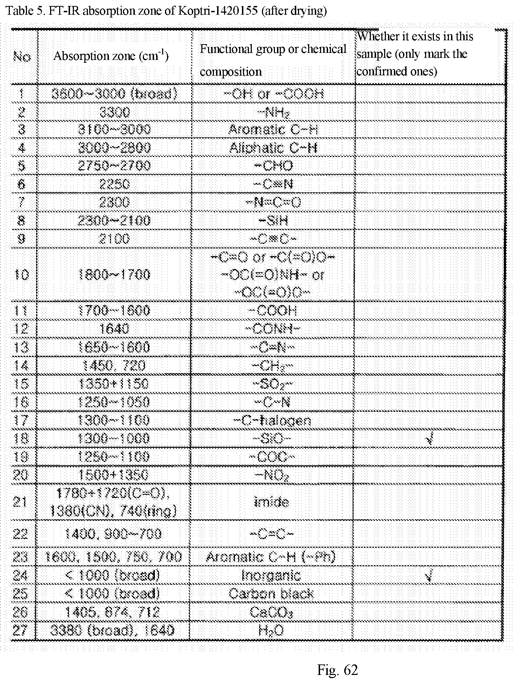

1. Entrusted institution: Korea Research Institute of Standards and Science
2. Name of sample: Koptri-1420155
3. Method of experiment: FT-IR, TGA, XRF, XRD
4.1. Result of experiment
   -- Analytical result of mineral somatid (name of sample: Koptri-1420155): as shown in Fig. 58, it is confirmed that the sample contains about 89% water, and does not contain specific organic components, calcium carbonate (CaCO₃), carbon black, etc. In addition, it is confirmed that the sample contains 7wt% SiO₂, 2wt% Al₂O₃, 1wt% Fe₂O₃, and 1wt% K respectively, besides water.
4-2 Result of Fourier Transform Ultra-Red Spectrophotometer (FT-IR) experiment
   (1) Measuring instrument: jasco ft-ir 4100
   (2) Measurement mode: Attenuated total reflection (ATR) mode
   (3) Resolution: 4cm⁻¹
   (4) Scna number: 32
   (5) FTIR Library List
      -- FT-IR analytical result: as shown in Figs. 60-62, it is confirmed that H₂O exists before the drying; and a component that has a spectrum very similar to the spectrum of SiO₂ exists after the drying.
4-3. Result of Thermogravimetric Analyzer (TGA) experiment
   (1) Measuring instrument: TA Instrument, Q500
   (2) Heating rate: 10°C/min
   (3) Temperature range: RT ∼ 800°C
   (4) Atmosphere: N₂
   (5) Result of experiment

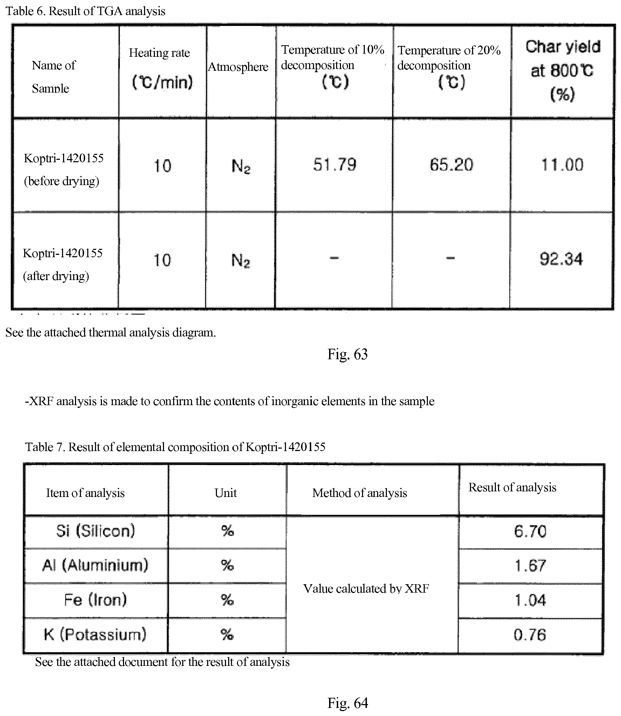

As shown in Fig. 63, compared with the char yield (11.00%) at 800°C before the drying, it is confirmed that the char yield at 800°C after the drying is 92.34%.
4-4. Result of X-Ray Fluorescence Spectrometer (XRF) experiment
   (1) Measuring instrument: Model 720 from Shimadzu
   (2) Atmosphere: Vacuum
   (3) Collimator: 10mm
   (4) Analysis mode: As indicated by the periodic table of elements Na∼U (Ti∼U, Na∼Sc), only the elements in the measuring range are set to 100% in the XRF experiment.
   (5) Result of experiment

As shown in Fig. 64, it is confirmed that the sample contains 6.70wt% SiO₂, 1.67wt% Al₂O₃, 1.04wt% Fe₂O₃, and 0.76wt% K respectively.

While some combinations or actions of all constituting elements of embodiments of the present invention are described above, the present invention is not limited to those embodiments. In other words, one or more of the constituting elements may be combined selectively and act, without departing from the scope of the present invention.

Furthermore, unless otherwise specified, the terms "comprise", "include", or "have", etc. in the above description mean including relevant constituting element, without excluding other constituting elements; rather, they should be comprehended as further including other constituting elements. In addition, unless otherwise defined, all terms, including technical or scientific terms, have the same meanings commonly understood by those having ordinary knowledge in the art.

The above description only describes the technical idea of the present invention exemplarily, and various modifications and variations can be made by those having ordinary knowledge in the art without departing from the scope of essential features of the present invention. Therefore, the embodiments disclosed in the present invention are not provided only to describe the technical idea of the present invention rather than confining the technical idea, and the scope of the technical idea of the present invention is not defined by the embodiments described above. The protection scope of the present invention shall be interpreted according to the attached claims, and all technical ideas within an equivalent scope shall be deemed as falling in the protection scope of the present invention.

### Industrial Applicability

According to the results of the experiments described above, the multi-functional natural gel or natural powder provided in the present invention is expected to attain effects including tissue necrosis prevention, skin regeneration, cancer cell inhibition, and increase of regulatory T cells, etc., and can be used in industrial fusion/composite materials to attain effects including arthritis or aging prevention, electromagnetic wave shielding and absorption, heat release, improvement of strength and elongation, anti-microbial function against coliform bacteria, staphylococci, pneumococci, and super bacteria, VOC removal, and water and air purification, etc.

## Claims

1. A method for manufacturing multi-functional natural gel and natural powder using mineral somatid, which is a method for manufacturing rehabilitating multi-functional natural gel and natural powder of quantum energy living body using mineral somatid powder extracted from minerals produced from the natural world, comprising:
a step of mineral collection: collecting minerals that contain components including SiO₂, Al₂O₃, Fe₂O₃, MgO₃, and K, etc., alone, or all of them;
a step of mineral milling: milling the minerals collected through the mineral collection step into 320-mesh or less mineral powder;
a step of heat treatment of residual organic substances: heating the mineral powder at 200∼1,000°C high temperature for 1∼2h, so as to remove impurities and residual organic substances;
a step of powder ripening: mixing the resultant mineral powder after the heat treatment of residual organic substances with water or distilled water at a certain weight ratio, and ripening and fermenting at normal temperature;
a step of ceramic powder separation: removing moisture content from the mixture after ripening and fermentation, and drying the mixture at 180°C for 3h or longer time after the moisture content is removed, so as to only separate mineral ceramic powder from the mixture;
a step of powder heating: reheating the ceramic powder at high temperature of 100∼1,000°C;
a step of mineral somatid separation: separating mineral somatid from the mineral ceramic powder after the reheating;
a step of natural gel manufacturing: heating and drying the mineral somatid at 100∼600°C for 1∼10h so as to evaporate moisture content, distilled water, and deionized water, and then manufacturing the mineral somatid into natural gel in a gel form; and
a step of natural powder manufacturing: treating the natural gel by heat treatment, to manufacture the natural gel into natural powder.

2. The method for manufacturing multi-functional natural gel and natural powder using mineral somatid according to claim 1, wherein,
in the step of mineral collection, the minerals are natural minerals including loess, medical stone, jade, germanium, tourmaline, zeolite, kaolin, bentonite, and crystal.

3. The method for manufacturing multi-functional natural gel and natural powder using mineral somatid according to claim 1, wherein,
the minerals are milled with alumina ball mill (alumina rotary mill) and jet mill through either a dry process or a wet process.

4. The method for manufacturing multi-functional natural gel and natural powder using mineral somatid according to claim 1, wherein,
the step of mineral milling further comprises a mineral powder sorting step, so that powders that contain SiO₂, Al₂O₃, Fe₂O₃, MgO₃, and K components alone, or all of them are sorted out respectively from the mineral powder after the milling.

5. The method for manufacturing multi-functional natural gel and natural powder using mineral somatid according to claim 1, wherein,
in the step of powder ripening, 10∼80wt% mineral powder is mixed with 20∼90wt% water or distilled water into a mixture, and then the mixture is stored in a specified container and ripened at 1∼100°C for 2∼60 days, and is fermented through the ripening.

6. The method for manufacturing multi-functional natural gel and natural powder using mineral somatid according to claim 1, wherein,
in the step of mineral ceramic powder separation, the mineral ceramic powder is separated from the mineral powder through a primary heating step and a secondary heating step,
in the primary heating step, the ripened mixture is loaded into a roasting oven and rotated at 180°C or a lower temperature for 30min.∼2h to remove the moisture content, and then heating the mixture in a desiccator at 100∼200°C for 3h to accomplish drying,
in the secondary heating step, which follows the primary heating step, the mixture is heated at 400∼1,000°C high temperature to accomplish drying.

7. The method for manufacturing multi-functional natural gel and natural powder using mineral somatid according to claim 1, wherein,
the step of mineral somatid separation comprises:
a stirring step: the mineral ceramic powder is mixed with water or distilled water or deionized water, and then the mixture is loaded into a stirring machine and stirred for 24h;
a mineral somatid separation step: after the stirring step is finished, the mixture is centrifuged in a centrifugal separator for 1∼24h, so as to accomplish centrifugal separation of the mineral ceramic powder and the mineral somatid;
a layer separation step: after the centrifugal separation is finished, precipitation is executed for 3∼72h, so as to accomplish layer separation of the mineral ceramic powder and water or distilled water or deionized water.

8. The method for manufacturing multi-functional natural gel and natural powder using mineral somatid according to claim 7, wherein,
in the layer separation step, the mixture is separated into a top layer portion, a bottom layer portion, and a middle layer portion,
the water or distilled water or deionized water is located in the top layer portion, mineral ceramic powder that fully contains components including SiO₂, Al₂O₃, Fe₂O₃, MgO₃, and K, etc., is located in the bottom layer portion,
the middle layer portion consists of a suspension and turbid liquid, in which the mineral somatid resides,
and the mineral somatid is separated by only collecting the middle layer portion that consists of the suspension and turbid liquid.

9. The method for manufacturing multi-functional natural gel and natural powder using mineral somatid according to claim 1, wherein,
the natural gel respectively contains 40∼80wt% SiO₂, 10∼35wt% Al₂O₃, 5∼10wt% Fe₂O₃, 3∼7wt% MgO₃, and 2∼8wt% K alone, or all of them.

10. The method for manufacturing multi-functional natural gel and natural powder using mineral somatid according to claim 1, wherein,
in the step of manufacturing natural powder of mineral somatid, the natural powder of mineral somatid is manufactured through a primary heat treatment step and a secondary heat treatment step,
in the step of primary heat treatment, the natural gel is dried at 100∼800°C for 2∼20h,
in the step of secondary heat treatment, the natural gel is treated by high pressure sterilization at 121°C for 15min. in an autoclave.

11. The method for manufacturing multi-functional natural gel and natural powder using mineral somatid according to claim 10, wherein,
in the step of manufacturing natural powder of mineral somatid, after the secondary heat treatment step, the resultant solid content obtained through the heat treatment is
further dried at temperature of 100∼800°C for 2∼20h.
